(19) Europäisches Patentamt
European Patent Office
Office européen des brevets

(11) **EP 3 751 473 A1**

(12) **EUROPEAN PATENT APPLICATION**
published in accordance with Art. 153(4) EPC

(43) Date of publication:
**16.12.2020 Bulletin 2020/51**

(51) Int Cl.:
***G06N 20/00*** *(2019.01)*      ***G06F 16/90*** *(2019.01)*
***G06Q 50/22*** *(2018.01)*

(21) Application number: **19751088.6**

(22) Date of filing: **12.02.2019**

(86) International application number:
**PCT/JP2019/004812**

(87) International publication number:
**WO 2019/156254 (15.08.2019 Gazette 2019/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.02.2018   JP 2018021518**
**16.05.2018   JP 2018094644**

(71) Applicant: **Axion Research Inc.**
**Tsukuba City,**
**Ibaraki 305-0047 (JP)**

(72) Inventors:
• **SATO, Tomoyoshi**
**Tsukuba-shi, Ibaraki 305-0047 (JP)**
• **SATO, Takumi**
**Tsukuba-shi, Ibaraki 305-0047 (JP)**

(74) Representative: **Körber, Martin Hans**
**Mitscherlich PartmbB**
**Patent- und Rechtsanwälte**
**Sonnenstrasse 33**
**80331 München (DE)**

(54) **SYSTEM THAT ESTIMATES STATE OF COMPLEX SYSTEM TO BE INSPECTED**

(57)      A system (10) that estimates a state of an individual complex system out of a multitude of target complex systems includes: storage (12) that stores matrices (13) of respective stages which include a large amount of correlation information for correlations between test results of a multitude of test items which suggest states of the multitude of target complex systems corresponding to stages in changes over time of the multitude of target complex systems; and a first estimating unit (21) configured to estimate a first state of a first complex system based on a first matrix (17) produced by converting test results for a multitude of test items at first timing of the first complex system that is an individual complex system into a matrix based on the large amount of correlation information for correlations between the test results of a multitude of test items in at least one matrix out of the matrices of the respective stages.

Fig. 1

**Description**

Technical Field

The present invention relates to a system that estimates the state of a target complex system from the test results of test items.

Background Art

Japanese Laid-open Patent Publication No. 2017-91586 discloses the provision of a health management server capable of generating appropriate advice messages based on the diet and state of health of a health management subject to increase motivation to perform a health management curriculum. The health management server disclosed in this document is connected via a network to a terminal owned by the target person of health management (the person health management subject), and includes: a receiver unit that receives, from the terminal, one piece of curriculum information that has been selected by the target person; a storage unit that stores behavior patterns of the target person; an analysis unit that analyzes into continued patterns where actions based on the curriculum information have been continuing and stalled patterns where actions based on the curriculum information have stopped; an artificial intelligence unit that predicts physical information and the appearance of the target person predicted in the future; a generating unit that generates the physical information and the appearance; and a transmission unit that transmits image information or example description information to the terminal.

Summary of Invention

There is demand for a system that can accurately estimate the states of complex systems, which are composed of many elements such as the human bodies (human beings), flora and fauna, meteorological phenomena, the human economies, engines, and manufacturing and/or producing plants, and whose respective elements are complexly and intricately intertwined, as target complex systems (inspection targets, systems to be tested, systems subject to testing).

One aspect of the present invention is a system that estimates a state of an individual complex system out of a multitude of target complex systems. This system (examination system or diagnosis system) includes: first storage that stores matrices (static matrices) of respective stages which include a large amount of correlation information for correlations between test results of a multitude of test items which suggest states of the multitude of target complex systems corresponding to stages in changes over time of the multitude of target complex systems; and a first estimating unit configured to estimate a first state of a first complex system that is an individual complex system based on a first matrix produced by converting test results for the multitude of test items at first timing of the first complex system into a matrix based on the large amount of correlation information for correlations between the test results of the multitude of test items in at least one matrix out of the matrices of the respective stages. The matrices of the respective stages include a plurality of cells including correlation information for correlations between test results of a plurality of test items. In the present specification, the expression "multitude" or "large number" is not a plural number including two or three, and indicates at least five or at least ten complex systems or test items being processed.

In this system, determinations about the state of a target complex system subject to testing are made not only using increases and decreases in the numerical values of test results of the test items (parameters) provided for determining specific abnormalities, but also use mutual relationships between a multitude or a large number of test items, that is, correlations between the test items as indices for determining the state of the target complex system. In addition, it may be believed that the correlations between the multitude of test items are not limited to a specific correlation suggesting a specific abnormality. For this reason, with the present system, a multitude of correlations between a multitude of test items of a multitude of target complex systems are acquired in advance. For each of a plurality of stages (a plurality of specific stages, static states) that have been specified due to the states of those stages being or being considered to be definitely identifiable out of a plurality of stages (states) to which the multitude of target complex systems transition due to changes over time (changes with the passage of time), the large amount of correlations of each stage of the multitude of the target complex systems are gathered together in a matrix (static matrix) including a plurality of cells, so that matrices of stage-by-stage (matrices of respective stages) are provided.

Examples of the respective stages over time of complex systems may include a normal state, various abnormal states (non-normal states, anomalous states) for which the causes have been established in advance, and a quasi-normal state which is neither a normal state nor an abnormal state. A quasi-normal state may be an abnormal state for which the cause has not been established. If specific quasi-normal states are known or confirmed from state displacements or transitions of a multitude of target complex systems, a state (stage) that is not included in the matrices of the respective stages, that is, the normal state, the abnormal states, and the quasi-normal states may be estimated as a new abnormal state or a new quasi-normal state. If the target complex systems are human beings or animals, the states of health may deteriorate over time (gradually or rapidly) from a favorable state due to various factors such as age, physical fitness, living environment, and disease, or may recover or improve with the passage of time due to treatment or changes in environment.

The first estimating unit may include a first AI unit configured to estimate the first state of the first complex system using artificial intelligence that has learned correspondence between the plurality of first matrices and the plurality of first states through machine learning. The artificial intelligence (AI) may be artificial intelligence produced by performing image recognition on the first matrix, which includes a plurality of cells, and learning the correspondence with the first states through machine learning. Each of the plurality of cells that constructs a matrix may be a two-dimensional or three-dimensional cell including correlation information on correlations between the test results of two or a plurality of test items. Typically, the cells may be two-dimensional cells in which information (correlation coefficients, correlation analysis) obtained by statistically processing the correlations between the test results of a first test item and the test results of a second test item is indicated using scatter diagrams, distributions, contour lines, color variations, and the like. Matrices including these cells can be analyzed and classified by image recognition using artificial intelligence. Accordingly, it is possible to accurately estimate the first state of the first complex system by converting the test results of the first complex system into an image with the matrices of the respective stages as a filter and performing image recognition with artificial intelligence.

This system may include a first generating unit configured to generate the first matrix that reflects relationships between test results of a plurality of test items at the first timing of the first complex system in each of the plurality of cells included in the matrices of the respective stages. By comparing the first relationships between the test results of the multitude of test items at the first timing of the first complex system subject to testing and the large amount of correlations between the multitude of test items of the matrices of the respective stages, it is possible to estimate a first state of the first complex system at the first timing.

This system may include a second generating unit configured to generate the first matrix where test results of a multitude of test items at the first timing of the first complex system are expanded into (laid out on, mapped on) the plurality of cells using a large amount of correlation information for correlations between the test results of the multitude of test items in the matrices of the respective stages. At each stage, if there are correlations between the test results, by using this information, it is possible to emphasize or edit the test results of the first complex system subject to testing in the first matrix, which makes it easier to estimate the first state more accurately.

The system may include an output unit configured to output the first matrix in which the plurality of cells are disposed in two dimensions with the multitude of test items set on X and Y axes. The first matrix, where a plurality of cells are disposed in two dimensions, can be visually grasped by humans as an image, and makes it possible for specialists to track and/or confirm paths and/or results estimated by this system, in particular by AI.

The system may further include a second estimating unit configured to estimate transitions in a state of the first complex system based on displacements between the first matrix at the first timing and a second matrix for the first complex system at second timing when time has passed from the first timing. The second matrix is a matrix produced by converting the test results of a multitude of test items of the first complex system subject to testing at the second timing to a matrix based on a large amount of correlation information for correlations between test results of the multitude of test items in at least one matrix out of the matrices of the respective stages.

The storage may include transition matrices (stage transition matrices) that include information on transitions in the large amount of correlation information between the matrices of the respective stages and reflect changes in state over time for the multitude of target systems. The second estimating unit may include a unit configured to compare displacements or transitions from the first matrix to the second matrix and transition matrices between stages to verify a second state of the first complex system that has been estimated based on the second matrix. In the transition matrices, the multitude of correlations may include information on transitions (displacements) from other stages, and may include transitions or displacements to other stages. In addition to static correlations between a multitude of test items, it is possible to compare changes over time of a multitude of test items with the transition matrices (dynamic matrices) indicating transitions between stages and thereby provide estimates of symptoms with greater accuracy.

The second estimating unit may include a unit configured to estimate transitions in the state of the first complex system from the second timing onward (transitions after the second point in time). The future progressions of the first complex system may be estimated based on a path (progression) in past changes over time of the first complex system.

The second estimating unit may include a second AI unit configured to estimate transitions in the state of the first complex system using artificial intelligence that has learned correspondence between displacements between the plurality of first matrices and the plurality of second matrices and changes in state over time in the multitude of target complex systems through machine learning. The second AI unit may be shared with the first AI unit or may be configured to use shared artificial intelligence. The second AI unit may also include a function that estimates the second state by performing image recognition on an individual displacement matrix produced by converting displacements over time (transitions with the passage of time) in the state of the first complex system to an image using dynamic matrices between stages as a filter.

It is desirable for the matrices of respective stages and the stage transition matrices to include as many test results as possible for the target complex systems. Test results of the first complex system whose state has been estimated by this system may be automatically incorporated as the information in these matrices. It is desirable for the number of test results for the target complex systems to be as high as possible and therefore this system may include a unit configured to automatically generate a large amount of correlation information (correlations) between a multitude of test items of each stage using replicas of the target complex systems. A "replica" may be a simulator that models the complex system or may be a model where test results are statistically obtained using random numbers.

The target complex systems may be the human bodies or any other complex systems that include a multitude of elements, such as animals, plants, manufacturing and/or producing plants, ships, vehicles, turbine engines, or the like. When the complex systems subject to examination are the human bodies, the system may function as a preliminary examination system for examinations by doctors.

Another aspect of the present invention is a method that estimates a state of an individual complex system out of a multitude of (a large number of) target complex systems using a computer. The computer includes first storage that stores matrices of respective stages which include a large amount of correlation information for correlations between test results of a multitude of (a large number of) test items which suggest states of the multitude of target complex systems corresponding to stages in changes over time in the states of the multitude of target complex systems. The matrices include a plurality of cells including correlation information for correlations between test results of a plurality of test items. The method includes causing the computer to execute a first estimating process that estimates a first state of a first complex system that is an individual complex system based on a first matrix produced by converting test results for the multitude of test items at first timing of the first complex system into a matrix based on the large amount of correlation information for correlations between the test results of the multitude of test items in at least one matrix out of the matrices of the respective stages.

The first estimating process may include estimating the first state of the first complex system using artificial intelligence that has learned correspondence between the plurality of first matrices and the plurality of first states through machine learning. Also, the method may include causing the computer to execute a process that generates the first matrix that reflects relationships between test results of a plurality of test items at the first timing of the first complex system in each of the plurality of cells included in the matrices of the respective stages. In addition, the method may include causing the computer to execute a process that generates the first matrix where test results of the multitude of test items at the first timing of the first complex system are expanded into the plurality of cells using a large amount of correlation information for correlations between the multitude of test items in the matrices of the respective stages.

The method may further include causing the computer to execute a second estimating process that estimates transitions in a state of the first complex system based on displacements with respect to the first matrix of a second matrix, which has been produced by converting test results of the multitude of test items at second timing when time has passed from the first timing, of the first complex system into a matrix based on a large amount of correlation information for correlations between test results of the multitude of test items in at least one matrix out of the matrices of the respective stages.

The storage may include transition matrices that include information on transitions in the large amount of correlation information between the matrices of the respective stages and reflect changes in state over time in the multitude of target systems. The second estimating process may include comparing displacements from the first matrix to the second matrix and the transition matrices and verifying a second state of the first complex system that has been estimated based on the second matrix. The second estimating process may also include estimating transitions in the state of the first complex system from the second timing onward.

Yet another aspect of the present invention is a program (program product) for executing the method described above using a computer. This program (program product) may be provided having been recorded on an appropriate recording medium, or may be provided via a network.

[Brief Description of Drawings]

[Fig. 1]

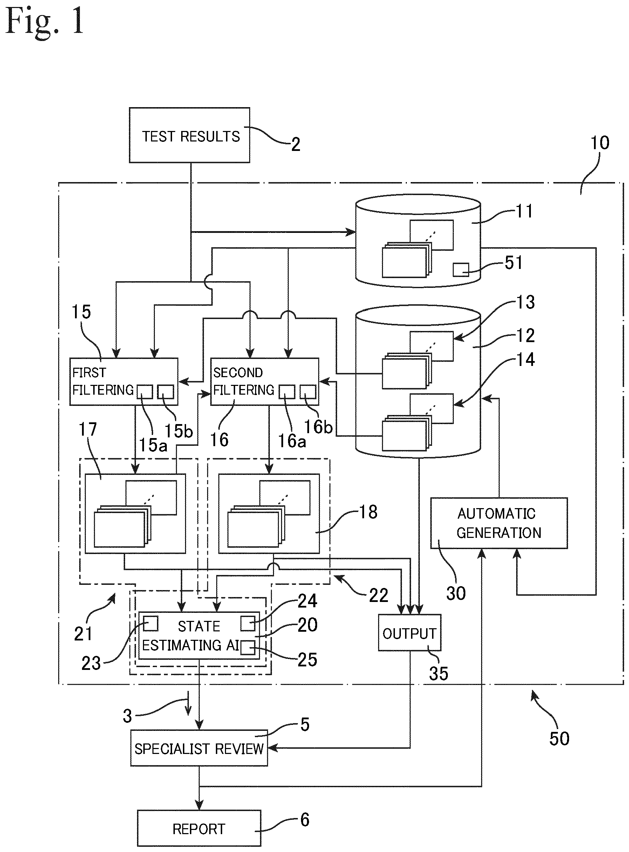

Fig. 1 is a block diagram depicting an overview of an examination system.
[Fig. 2]

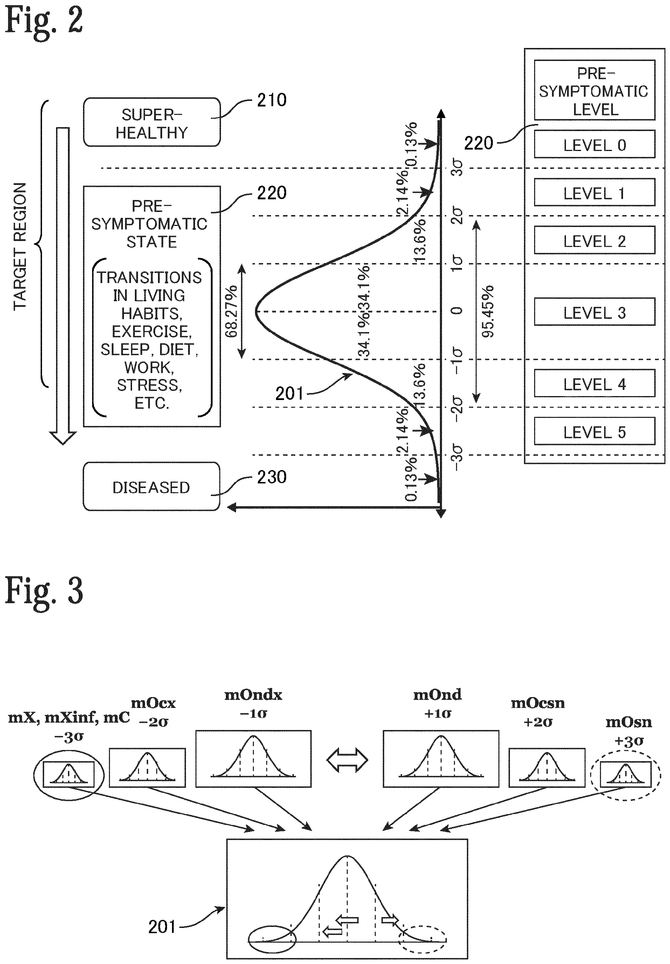

Fig. 2 is a diagram depicting one example of stages where the health states of patients, as one example of complex systems, change over time.
[Fig. 3]
Fig. 3 is a diagram schematically depicting statistical data of healthy, presymptomatic, and diseased patients.
[Fig. 4]

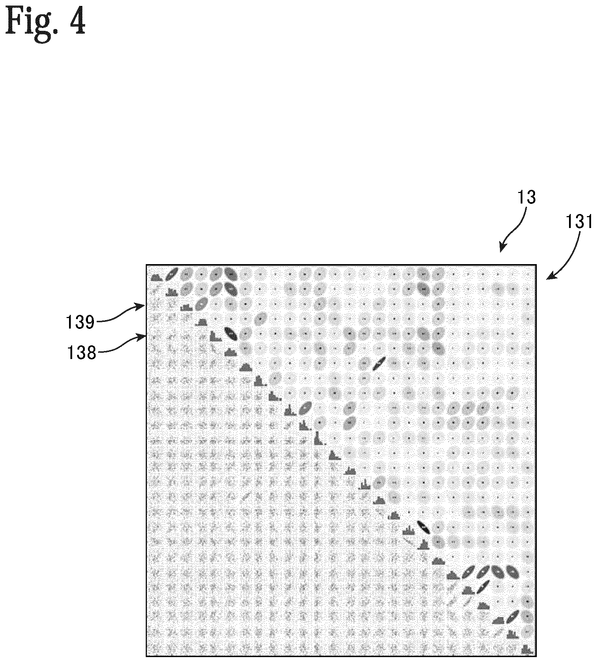

Fig. 4 is a diagram depicting one example of matrices at the super-healthy stage, as one example of matrices of respective stages (static matrix).

[Fig. 5]

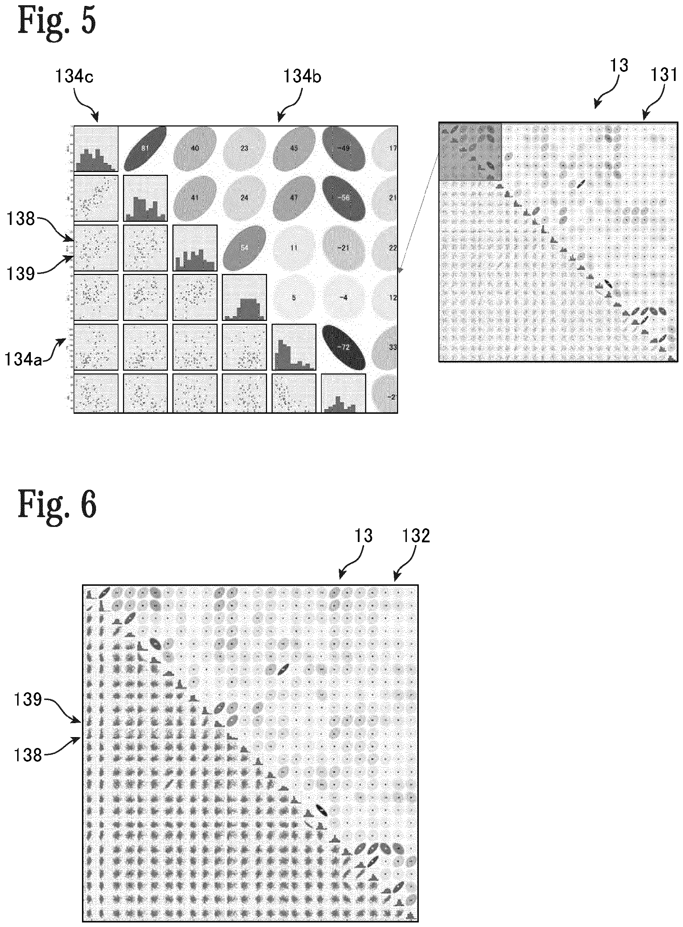

Fig. 5 is an enlargement of a part of the matrix depicted in Fig. 4.

[Fig. 6]

Fig. 6 is a diagram depicting one example of matrices at the presymptomatic stage, as one example of matrices of respective stages.

[Fig. 7]

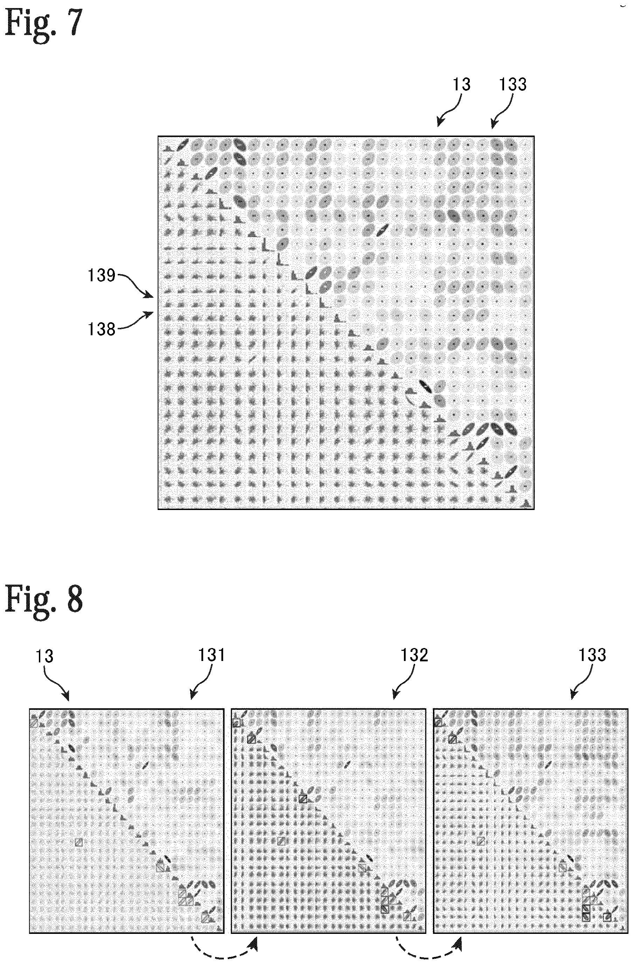

Fig. 7 is a diagram depicting one example of matrices at the diseased stage, as one example of matrices of respective stages.

[Fig. 8]

Fig. 8 is a diagram depicting a comparison of the matrices of a plurality of stages.

[Fig. 9]

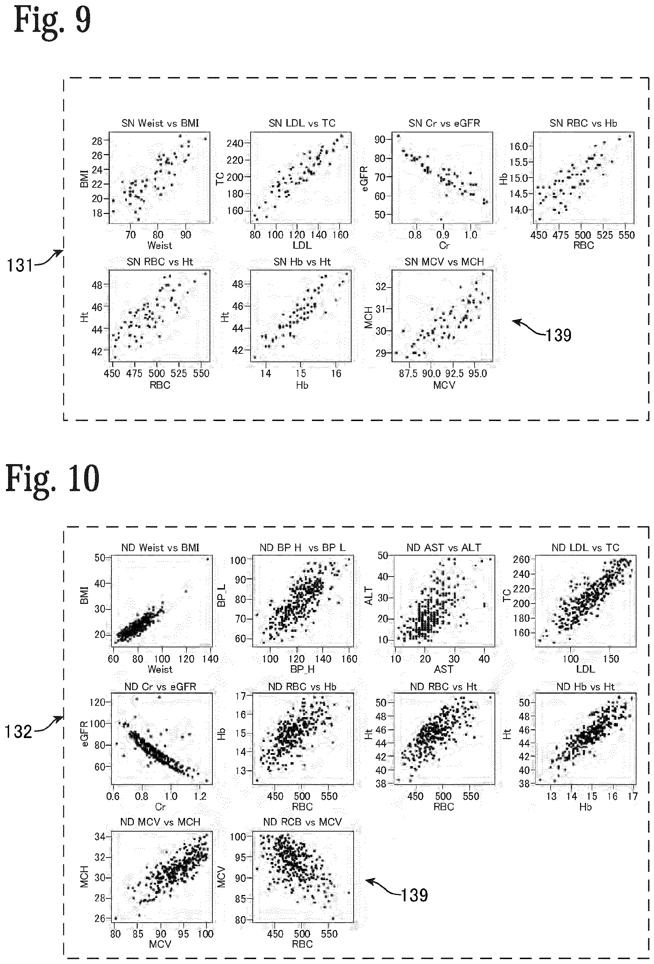

Fig. 9 is a diagram depicting a number of example scatter diagrams included as cells in the matrix for the super-healthy stage.

[Fig. 10]

Fig. 10 is a diagram depicting a number of example scatter diagrams included as cells in the matrix for the presymptomatic stage.

[Fig. 11]

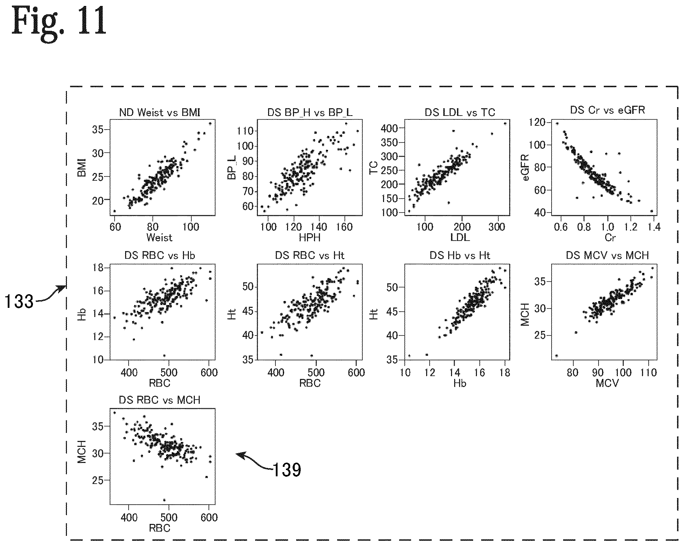

Fig. 11 is a diagram depicting a number of example scatter diagrams included as cells in the matrix for the diseased stage.

[Fig. 12]

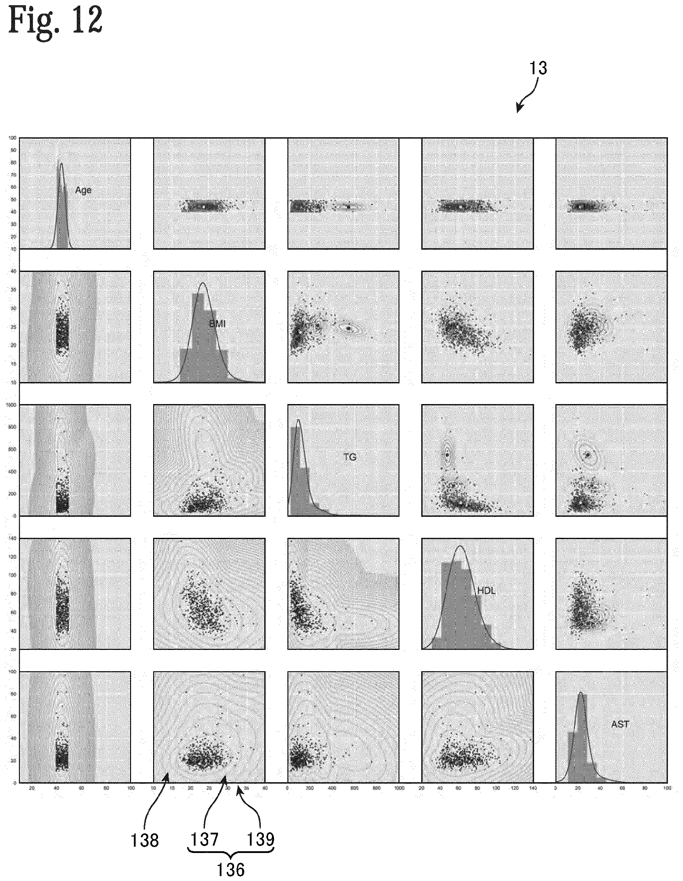

Fig. 12 is a diagram depicting different examples of the matrices for each stage.

[Fig. 13]

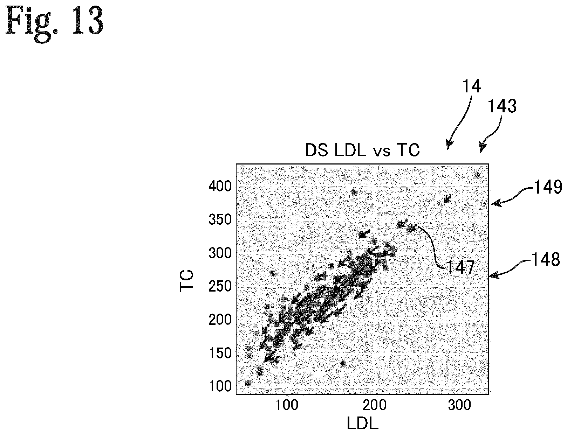

Fig. 13 is a diagram depicting an example of transition matrices (vector map).

[Fig. 14]

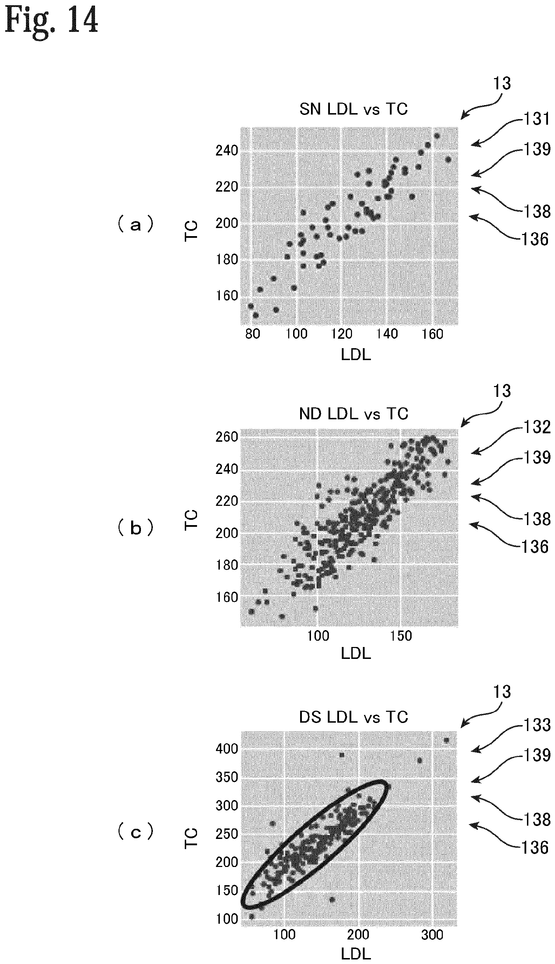

Fig. 14 is a diagram depicting how correlations included in the cells of the matrices of respective stages change depending on the stage, where, as examples, Fig. 14(a) depicts the super-healthy stage, Fig. 14(b) depicts the presymptomatic stage, and Fig. 14(c) depicts the diseased stage.

[Fig. 15]

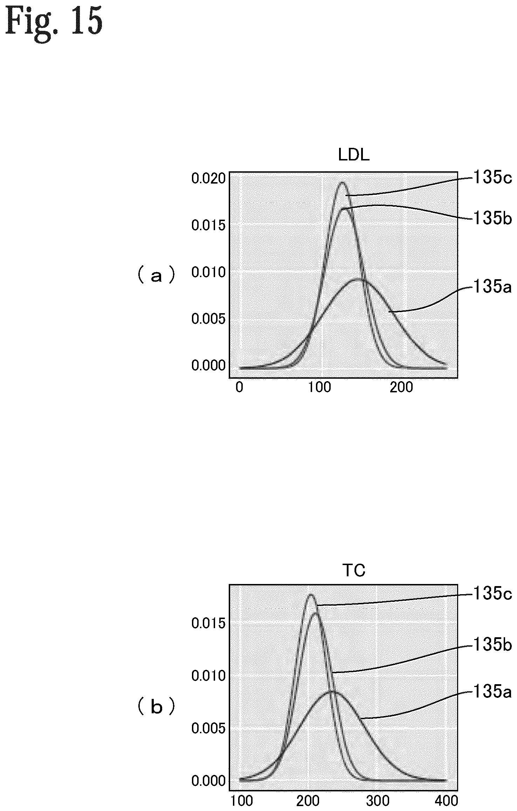

Fig. 15 is a diagram depicting how the distributions of test results of test items change at respective stages, with Fig. 15(a) depicting the distribution of LDLtest results and Fig. 15(b) depicting the distribution of TC test results.

[Fig. 16]

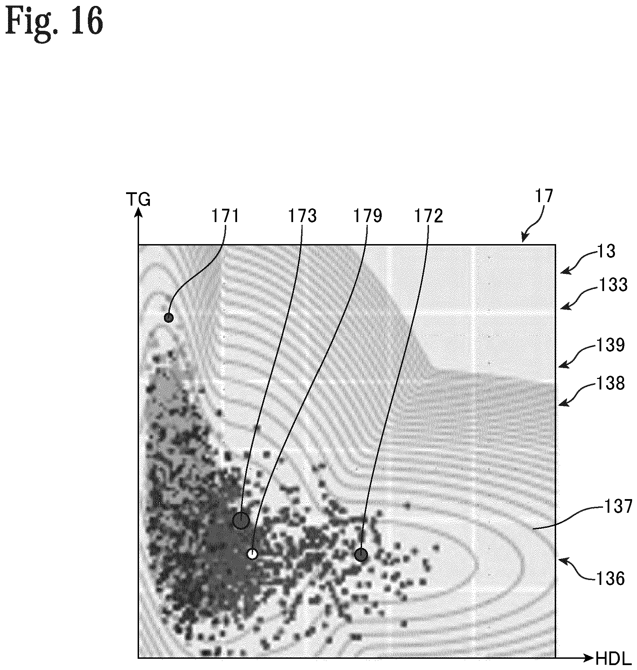

Fig. 16 is a diagram depicting an example of a cell included in an individual matrix.

[Fig. 17]

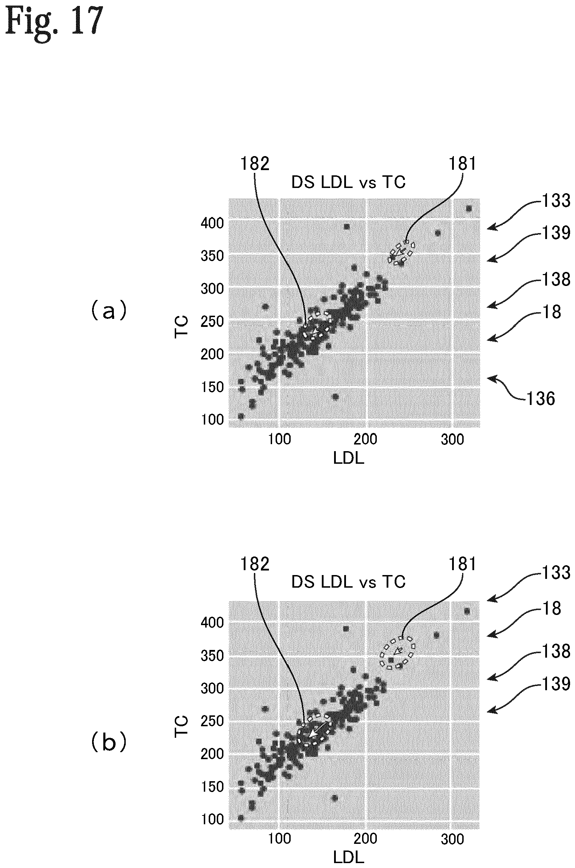

Fig. 17 is an example of cells included in an individual displacement matrix (individual transition matrix), with Fig. 17(a) depicting an example where displacements of the test results of a patient are reflected in the cells of a static matrix and Fig. 17(b) depicting an example where displacements are represented using correlation information.

[Fig. 18]

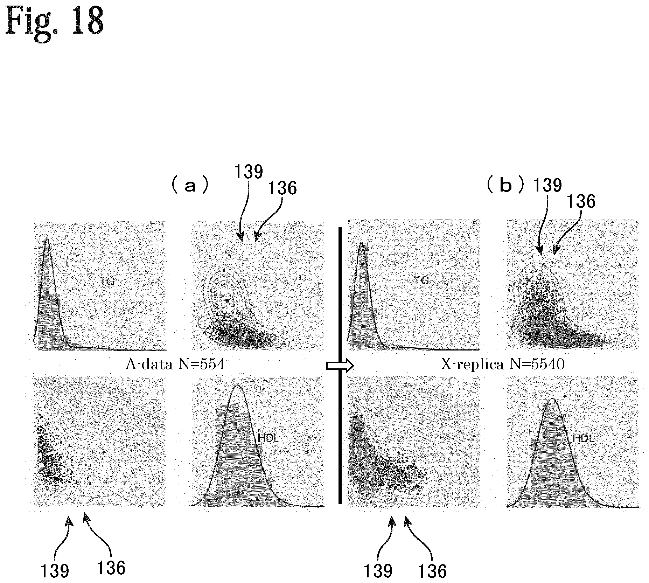

Fig. 18 is a diagram depicting an example of automatic generation of matrices, with Fig. 18(a) depicting an example of correlation information produced using real samples and Fig. 18(b) depicting an example of correlation information produced using replicas.

[Fig. 19]

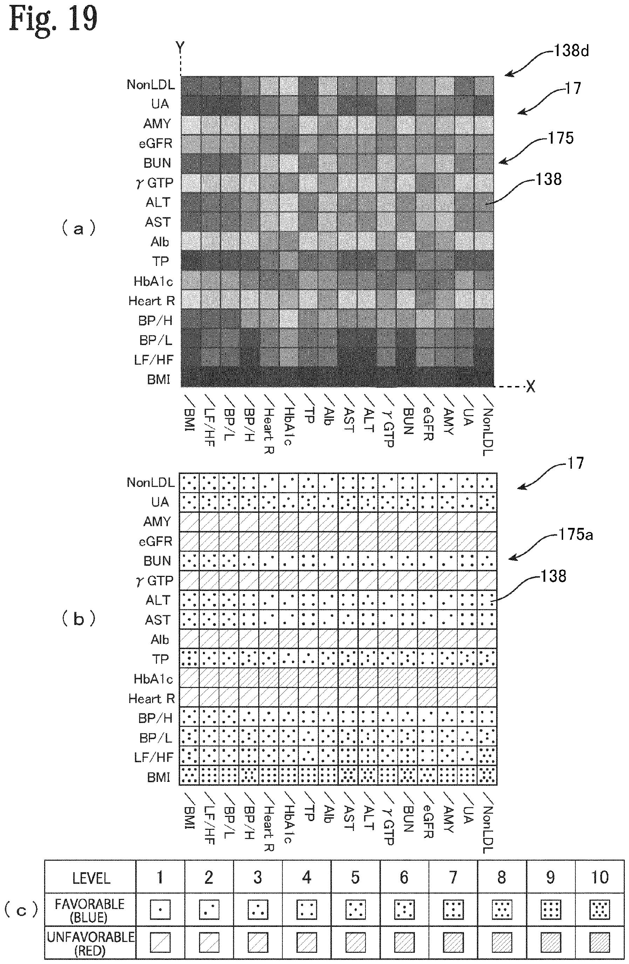

Fig. 19 is a diagram depicting an example of a heat map which is another example of individual matrices, with Fig. 19(a) depicting the heat map using shades of colors and Fig. 19(b) as a diagram produced by replacing shades with symbols in accordance with a correspondence table depicted in Fig. 19(c).

[Fig. 20]

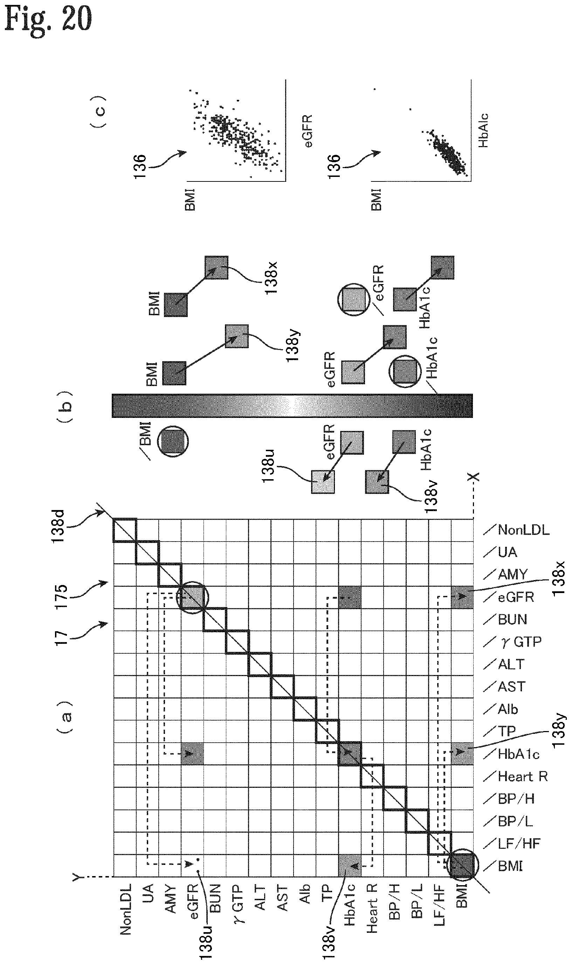

Fig. 20 is a diagram depicting a method of generating a heat map, with Fig. 20(a) depicting how information in cells on a diagonal is expanded and Fig. 20(b) depicting how deviation values of cells on a diagonal are shifted using correlation coefficients depicted in Fig. 20(c).

[Fig. 21]

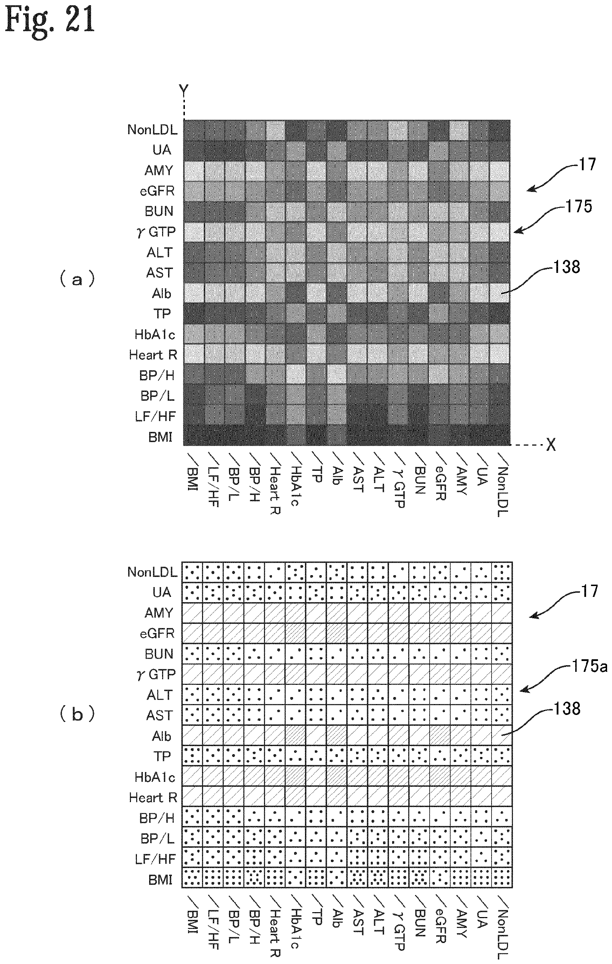

Fig. 21 is an example of a heat map for when time has passed from the heat map of Fig. 19, with Fig. 21(a) depicting the heat map using shades of colors and Fig. 21(b) depicting the heat map using symbols corresponding to the shades.

[Fig. 22]

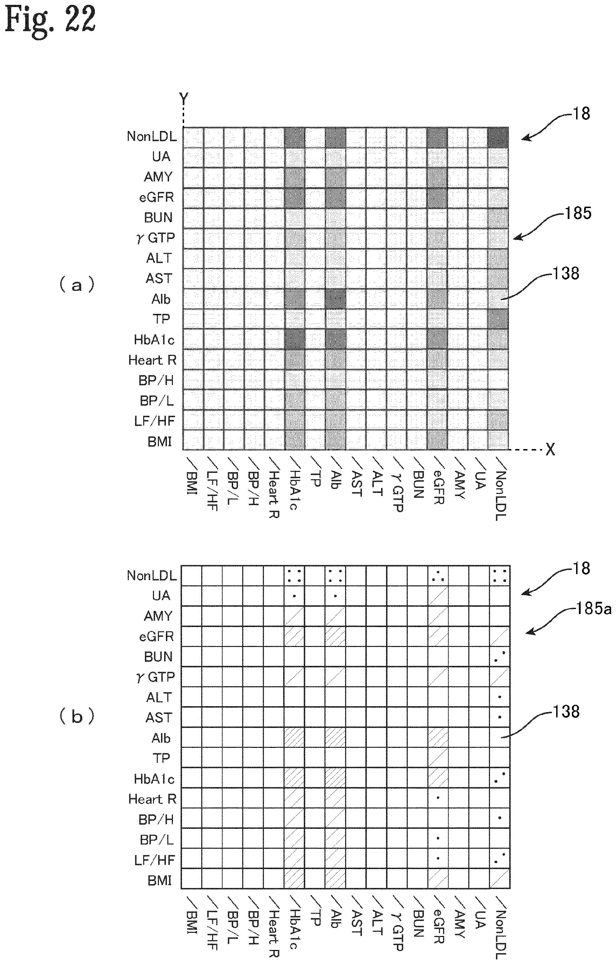

Fig. 22 is an example of a heat map depicting the differences between the heat maps depicted in Figs. 19 and 21, where Fig. 22(a) depicts a differential heat map using shades, and Fig. 22(b) depicts a differential heat map using symbols corresponding to the shades.

[Fig. 23]

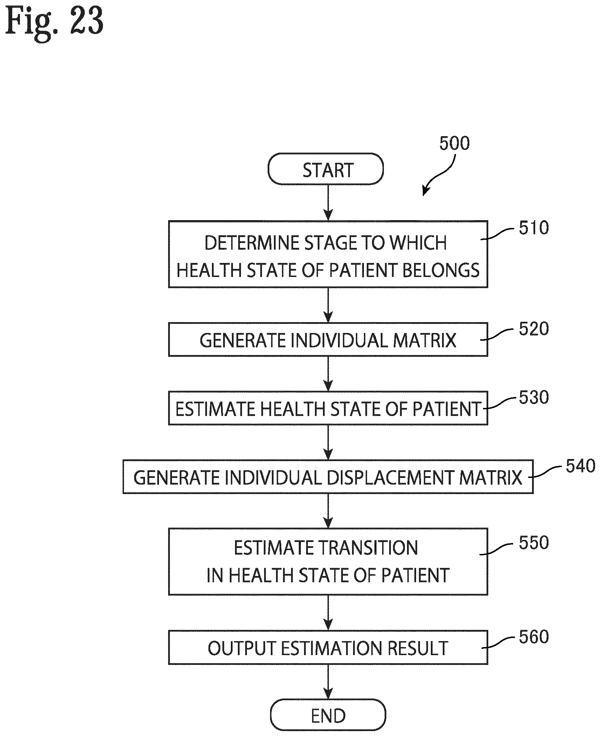

Fig. 23 is a flowchart depicting an overview of processing of an examination system.
[Fig. 24]

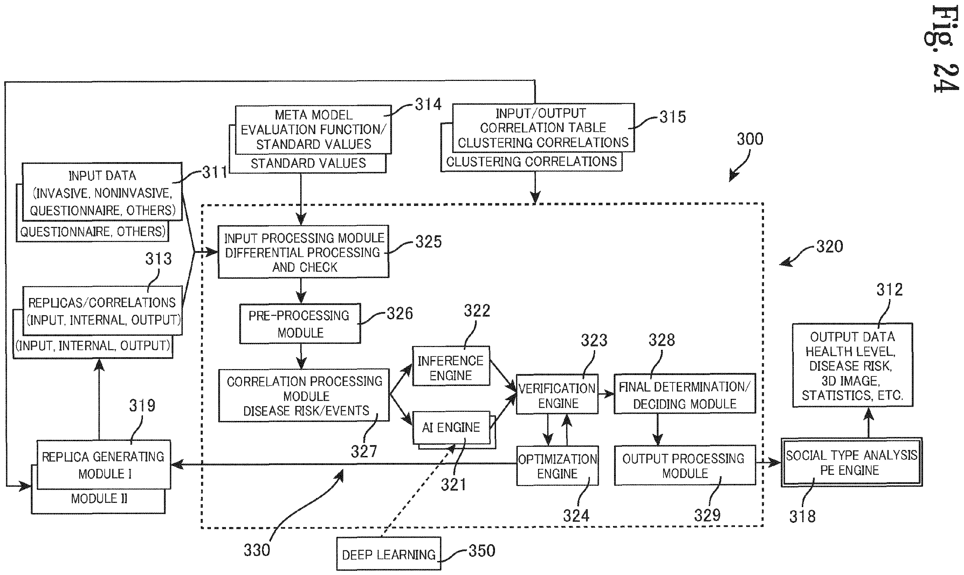

Fig. 24 is a block diagram depicting an overview of a diagnosis engine equipped with a hybrid engine.

Description of Embodiments

Fig. 1 depicts an overview of a preliminary examination system where the human bodies (humans or persons) are the target systems subject to testing. This preliminary examination system (pre-examination system, examination system, diagnostic system, diagnostic device, or system) 10 outputs examination results (including the health state of a patient that has been estimated or diagnosed) based on test results (inspection results) 2 for the person (patient) who is the target complex system, and submits a report 6 to the patient after undergoing a review by a doctor 5, who is a specialist. One example of the examination system 10 is realized by a computer that executes a program (program product) for an examination system. The examination system 10 includes a library (storage) 11 that stores past and present test results 2 for respective patients, a program 51, and the like, and a library (first storage) 12 that stores static matrices 13 that are matrices of respective stages (matrices for each stage or matrices of stage-by-stage) and matrices 14 indicating transitions between stages (transition matrices, stage transition matrices, or dynamic matrices).

It is known that due to changes over time, the state of a complex system transitions from a normal state to an abnormal state (unnormal state) by way of a quasi-normal state which is not a normal state, but is not an abnormal state. For a human being as an example of a complex system, the body changes from a super-healthy state (normal state) to a diseased state (abnormal state or non-normal state) through a state called "presymptomatic" ("mibyou" in Japanese, presymptomatic state, pre-disease, undiagnosed, quasi-normal state).

Fig. 2 depicts the relationship between super-healthy, presymptomatic, and diseased. For health care, when measurement data (test results) for a blood test or urine test are within normal values, according to conventional diagnostic standards this means that everything is normal and there are no particular problems. That is, if some measurement data is within normal values, the subject is determined to be healthy with regard to diseases that are related to that measurement data, and if all the measurement data being measured are within the ranges of normal values, there is no danger regarding diseases and the subject (the target system) is recognized as being super-healthy.

However, the inventor of the present application proposes that from the viewpoint of managing future risks including the possibility of transitions to specific diseases, it is necessary to analyze weak correlations between test results of test items. By confirming correlations between test results (input data), there is the possibility that relevance may appear for a plurality of data relating to a specific organ inside the human body, for example. By expressing these correlations as a matrix of a plurality of clustered data, it may be possible to analyze the distance from a super-healthy body as a deviation value (from the standard deviation). By performing statistical processing of correlation between test results of a variety of test items (such as age, body condition, bone density, muscle mass, fat, blood vessel age, capillary activity estimated for the body, immunoreactivity, HLA, DNA, microRNA, exosomes, and the like) at each transitional stage and/or together with other conditions and comparing with the test results for individuals, it is possible to estimate or predict nodes (change points or branching points to a plurality of diseases) where there is a shift from healthy via presymptomatic to diseased. Based on this, by creating disease growth maps (disease trees) until respective diseases are reached and tracing movements on these maps, it becomes possible to predictively estimate the risks of diseases occurring in the future.

As depicted in Fig. 2, as changes over time in the human bodies (persons), it is possible to define states in stages from super-healthy 210 via presymptomatic 220 to diseased 230. In addition, regarding presymptomatic ("mibyou") 220, when it is assumed that the distribution of health states of certain people in the population 201 is a normal distribution, it is possible to set levels from "level 1" (where level 0 is super-healthy 210) to "level 5" as described below.

Level 0: Super-healthy

"Super-healthy" refers to a healthy person who at present has no known signs of disease risk. There are statistics that indicate that only a few percent of people who take medical checkups can be referred to as "super-healthy" or "super-normal".

Level 1: "Edge of Presymptomatic" or "Almost Healthy"

This level indicates subjects where compared to super-healthy, the standard deviation value is in a range of 45 to 50, and one or more marker substances and/or combinations of input values indicating one or more diseases have been detected. When subjects do not qualify as Level 2, they are assumed to be Level 1.

Level 2: "Start of Transition to Presymptomatic" or "Unhealthy State"

This level is where the standard deviation value is in a range of 40 to 50, and two or more marker substances and/or combinations of input values indicating one or more diseases have been detected. This level is demarcated from Levels 1 and 3.

Level 3: "Typical Presymptomatic State" or " Disease Risks Coming out"

This level is where the standard deviation value is in a range of 35 to 45 and three or more marker substances and/or combinations of input values indicating one or more diseases have been detected. This level is demarcated from Level 4.

Level 4: "Presymptomatic State Requiring Attention" or "Advise Disease Risks"

This level is where the standard deviation value is in a range of 30 to 40, and four or more marker substances and/or combinations of input values indicating one or more diseases have been detected. When possible in this state, it may be advisable to undergo a detailed examination at a medical institution. This level is demarcated from Level 5.

Level 5: "Clear Transition from Presymptomatic to Diseased State" or "Disease Risks Warning"

This level is where the standard deviation value is within the range of 20 to 35 and five or more marker substances and/or combinations of input values indicating one or more diseases have been detected. There is the possibility that the disease will have progressed to a risk area identified by a medical institution as a specific disease or complication. In this case, it is better to undergo a detailed examination without delay.

Fig. 3 depicts changes over time of the population 201 in various stages based, for example, on immunity type (HLA), immune system activity (number and activity of immune cells), and other various parameters for maintaining basic health. From the right side, there is a shift from the super-healthy 210 (super normal mOsn) 210 to diseased 230 through stages such as healthy (mOcsn, presymptomatic level 1), presymptomatic A (almost healthy: mOnd, presymptomatic level 2 or 3), presymptomatic B (almost diseased: mOndx, presymptomatic level 3 or 4), onset of disease (disease onset: mOcx, presymptomatic level 5), complications (various complications: mCx), infected (various types: mXinfx), and diseased (diseased ds: MXooo). In addition, when people who correspond to each stage are extracted, the state (state of health) of the population of the extracted people will also have some kind of distribution, for example, a normal distribution, within each stage. Accordingly, depending on what position in the distribution of respective stages corresponds to the states of health of a subject, it is possible to judge, within the presymptomatic levels 1 to 5, whether the subject is stable, is likely to deteriorate, or is likely to improve, for example.

As one example, at present, blood test data is normally determined to be normal or healthy if the respective test items independently fall within ranges based on the standard values of people who are statistically considered to be healthy through health examinations and other tests conducted by medical institutions. However, an experienced doctor may believe there is a problem even if he or she notices that there are a number of test items which, while not completely out of the standard range, are exhibiting a tendency where the difference with standard values is deteriorating. It is known that the timing of a shift from healthy to presymptomatic and a further shift to diseased will vary depending on basic parameters corresponding to age and health and also immune activity. In the present invention, super-healthy patients, where it has been confirmed from normal healthy bodies that there is little correlation between data, are actively and intentionally extracted from actual measurement data. This data is used as reference data for super-healthy, and shifts from the super-healthy state 210 through the presymptomatic stage 220 to a variety of diseased stages 230 can be estimated by referring to correlation information for test results for a multitude of test items, such as: age; body condition; bone density, muscle mass, fat, blood vessel age, capillary activity assumed inside the body; immunoreactivity; HLA; DNA; microRNA; and exosomes, and/or analysis results of them. In addition, it is possible to generate disease development maps (disease trees) that reach various diseases from nodes (change points or branching points to multiple diseases) where there are shifts from healthy via presymptomatic to diseased. By tracing movements, it becomes possible to predictively estimate the risk of future diseases.

Fig. 4 depicts one example of static matrices 13 that are matrices for respective stages. Fig. 5 depicts an enlargement of part of this static matrix 13. The shown static matrix 13 is an example of a static matrices 131 for a normal state (super-healthy). Static matrices 13 are collections of correlation diagrams created from statistical data of test results that use, as a population, a large number of people (a multitude of target systems subject to testing) such as people of each race, each gender, or each specific region where a target patient is included, or alternatively the entire human race. One (one type of) static matrix 13 represents one of several situations and states that humans included in the population may pass through. A plurality of static matrices 13 are provided for a plurality of states respectively in the library 12. The static matrix 131 for a normal state is an example of a result of analyzing the correlation between statistical data of test results of patients determined to be normal in a medical examination or the like, out of the population to which the patient belongs.

The static matrices 13, which are matrices for each stage, each include a large amount of correlation information for correlations between the test results of a multitude of (a large number of) test items for a multitude of (a large number of) patients who form a population to which the patient (subject) belongs. In more detail, each static matrix 13 includes a plurality of cells 138 including correlation information for correlations between the test results of a plurality of test items out of a large number of test items. Each cell 138 contains correlation information obtained by statistical processing of the test results (measurements) of each test item (parameter) for respective individuals in a large number of patients. In the static matrices 13 in this example, each cell 138 is two-dimensional, and depicts the correlation between the test results of two test items out of the large number of test items using a scatter diagram 139. That is, a static matrix 13 in the present example is a collection (matrix) of a large number of scatter diagrams 139 depicting correlations between test items where the test results of respective test items for individuals in a large number of patients have been plotted with the X axis and the Y axis as coordinates so as to depict the correlation between the test items.

Note that the correlation information included in the cells 138 is not limited to the two-dimensional scatter diagrams 139 where numerical values (test results) of two different test items out of a large number of test data are plotted as X and Y coordinates, and may be correlation in three dimensions or multi-dimensional correlation. In place of scatter diagrams, information such as the presence or absence of correlation and correlation coefficients (including positive and negative coefficients) obtained using scatter diagrams may be depicted using numerical values, colors, patterns, graphs, or the like. The static matrices 13 are not limited to having the cells 138 arranged in two dimensions, and may be three-dimensional arrays or n-dimensional arrays. As one example, cells 138 that include numerical values or correlations for a large number of test items may be indicated by multi-dimensional positions (relationships) that have of each of the multitude of test items as coordinates. Static matrices 13 where the cells 138 are arranged two-dimensionally are one form of information that is easy for human experts such as doctors to review. In the present specification, a "matrix" or "matrices" are collections (maps or arrays) where a large number of "cells" (a unit capable of expressing statistical information and/or correlation information for one or a plurality of values on a plane or in a space that is two-dimensional or multi-dimensional with three or more dimensions) are arranged in two dimensions or three or more dimensions.

A multitude of test items (a large number of test contents) included in each static matrix 13 may include measured values of blood tests, urine tests, breath/skin gas measurements, body temperature, visceral fat, subcutaneous fat, muscle mass, body fat percentage, estimated bone mass, blood pressure, blood flow, heart rate, autonomic nerve excitement, and the like; image diagnosis results; microRNA, ncRNA, and DNA; and also medical questionnaires. Such measured values obtained in tests (here, "test results") are used in the scatter diagrams 139. As examples, TG (triglyceride, neutral fat), HDL (HRL cholesterol), LDL (LDL cholesterol), FBS (glucose (fasting blood sugar)), HbAlc (hemoglobin A1c), AST (aspartate aminotransferase), ALT (alanine aminotransferase), gGT (y-glutamyl transferase), ALP (alkaline phosphatase), LD (lactate dehydrogenase), TP (total protein), TC (total cholesterol), BUN (blood urea nitrogen), Cr (creatinine), eGFR (estimated glomerular filtration rate), UA (uric acid), RBC (red blood cell count), Hb (hemoglobin), Ht (Hematocrit), MCV (mean corpuscular volume), MCH (mean cell hemoglobin), and MCHC (mean corpuscular hemoglobin concentration) can be given as examples of measured items in a blood test.

The static matrix 13 depicted in Fig. 4 and Fig. 5 as an example has scatter diagrams 139 for a large number of test items, which include the test items described above and other items, for example, physical or cardiopulmonary test items such as waist measurement, BMI, BP_H (highest blood pressure), and BP_L (lowest blood pressure), for patients determined to be super-healthy arranged two-dimensionally to facilitate image recognition. In this static matrix 13, to illustrate the correlations between test items, all the numerical values are plotted as points in respective cells 138 in the triangular area 134a at the lower left, and the correlations between the test items are expressed graphically and numerically in the cells 138 of the triangular area 134b at the upper right. When correlation is hardly observed, this is indicated by low numbers, with "+" values indicating positive correlation and "-" values indicating negative correlation. It can be understood that there is a large correlation between some test items. In the static matrix 13, positive/negative and the magnitude of each correlation coefficient of the respective cells 138 are depicted by graphical elements (shapes, the inclination and elliptical forms) and shades of colors in respective cells 138 in the triangular range 134b at the upper right. Note that the cells 138 disposed on the diagonal line 134c, which is the boundary between the triangular ranges, indicate the distributions in each stage of the test results for each test item.

Fig. 6 depicts another example of static matrices 13. Any number of matrices 13 which include a plurality of cells 138 including correlation information, can be generated by changing the conditions of the population of target group. Here, for each stage in the changes over time, that is, for super-healthy, the respective levels of presymptomatic, diseased, and the like, matrices whose populations are target groups at the respective stages are generated as matrices 13 for static states (static matrices).

The static matrix 13 depicted in Fig. 6 is an example of a static matrix 132 in a quasi-normal (presymptomatic, "mibyou") state, and is one example of the result of analyzing correlations in statistical data of test results for a large number of patients, out of the population to which the patient belongs, who were not judged to be "normal" or "unnormal (abnormal)" in a medical examination or the like. The expression "presymptomatic ("mibyou" in Japanese)" refers to people left in the measurement data/input data group after super-healthy and diseased people have been excluded. The expression "super-healthy" refers to patients who exhibit normal values in all items (measurement data, input data, and medical interviews) in a clinical or medical checkup, and the expression "diseased" refers to patients who have been determined to be ill by a doctor. "Presymptomatic" will normally be the state to which the largest number of patients are likely to belong. It is possible to define states in a plurality of stages from a state that is close to healthy to a state close to diseased, and to provide heat maps for respective states.

Fig. 7 depicts yet another example of static matrices 13. The static matrix 13 shown in Fig. 7 is an example of the static matrices 133 for an abnormal (diseased) state and is an example of the result of analyzing correlations in statistical data on test results of a large number of patients who have been determined by doctors to have one or more specified diseases, out of the population to which the patient belongs. It is possible to provide static matrices 133 for diseased for respective stages of progression of respective specific diseases.

In Fig. 8, matrices 13 of the respective stages, that is, a static matrix 131 for super-healthy, a static matrix 132 for presymptomatic, and a static matrix 133 for diseased are disposed side by side. Fig. 9 depicts a number of examples of typical correlation diagrams 139 included in the static matrix 131 for super-healthy, Fig. 10 depicts a number of examples of typical correlation diagrams 139 included in the static matrix 132 for presymptomatic, and Fig. 11 depicts a number of examples of typical correlation diagrams 139 included in the static matrix 133 for diseased. Hardly any correlation is identified in the typical correlation diagram 139 for super-healthy body included in Fig. 9 and the plot is scattered. The correlation diagrams 139 included in Fig. 10 indicate that unlike super-healthy, there are some test items where clear correlation is identified. The correlation diagrams 139 included in Fig. 11 indicate that there are more test items where the same level as presymptomatic or stronger correlation is identified.

These figures depict a part of examples, and since there are different numbers of patients at each stage, there are fluctuations in the number of measurement values in the scatter diagrams 139 included in each matrix 13. As the number of samples included in the population increases, the characteristics of each stage should become clearer in the matrices 13 for respective stages. As can be understood from these matrices 13, at each stage (in each state), there are increases and decreases in the test items identified as having strong correlations, and it can be understood that the static matrices 13 indicating correlations between a large number of test items will differ at each stage. Accordingly, by comparing these static matrices 13 with relationships (first relationships) between a multitude of test items included in the test data of each patient, it is possible to estimate the state of each patient at the time of testing (first timing). In addition, it is possible to evaluate the test results of each patient by using the static matrices 13 of respective stages as filters and thereby estimate the state of each patient.

Fig. 12 depicts another example of a static matrix 13. Each cell 138 of the static matrix 13 includes a scatter diagram 139 and a probability distribution 137 indicating correlations in the scatter diagram as correlation information 136. Each cell 138 includes a scatter diagram 139 and contour lines indicating regions that have different statistical probabilities (standard deviations). According to the inventor of the present application, cases where the correlation between the test data of two test items provided in a scatter diagram 139 can be expressed by a single normal distribution and cases where the correlation needs to be expressed as a mixture of a plurality of normal distributions have been found. When the correlation is depicted as a mixture of a plurality of normal distributions, these normal distributions may be mixed on a one-to-one basis, or may be mixed at different ratios. By expressing the correlation between test items as a probability distribution, as described later, it is possible to use the static matrices 13 as filters for visualizing the relationships between the test items of each patient. In addition, when the number of statistical data is small, a statistically significant number of samples may be virtually generated from replicas to generate the static matrix 13, or some or all of the scatter diagrams 139 of the static matrix 13 may be supplemented.

Fig. 13 depicts one example of transition matrices (dynamic matrices) 14 that include transitions between stages for the static matrices 13 indicating correlations at each stage; that is, the transition matrices 14 include transitions in the correlation information (dynamic information) between the static matrices 13 of respective staves. Fig. 13 is one example of a plurality of cells 148 included in the transition matrices (vector maps) 14, and depicts a vector-appended scatter diagram 149 included in the transition matrix 143 for diseased (non-normal). The scatter diagram 149 shown in Fig. 13 depicts the correlation between the total cholesterol (TC) and low-density lipoprotein (ND LDL) out of the test items. The vector map 14, which is one example of a transition matrix, includes displacements (transitions) in a multitude of correlations between the static matrices 13 of the respective stages, and reflects changes in the state over time in the population that includes the patient. The vector map 14 in this example indicates, as a cell 148 showing displacements in correlation information, arrows (vectors) 147 indicating the directions and magnitudes of changes over time in the scatter diagram 149.

Fig. 14 depicts scatter diagrams 139 including the correlation information 136 of total cholesterol (TC) and low-density lipoprotein (ND LDL) as an examples of the cells 138 included in the static matrix 13 at each stage. Fig. 14(a) depicts a scatter diagram 139 included in the matrix 131 of the super-healthy stage, Fig. 14(b) depicts a scatter diagram 139 included in the matrix 132 of the presymptomatic stage, and Fig. 14 (c) depicts a scatter diagram 139 included in the matrix 133 of the diseased stage. Fig. 15(a) depicts the distribution of measured values of low-density lipoprotein (ND LDL) at each stage, and Fig. 15(b) depicts the distribution of measured values (test results) of total cholesterol (TC) at each stage.

First, as shown in Figs. 15(a) and (b), the distribution 135a for super-healthy has a curve with a low peak, the distribution 135c for diseased has a curve with a high peak, and the distribution 135b for presymptomatic is in between. Accordingly, the correlation between total cholesterol (TC) and low-density lipoprotein (ND LDL) changes (i.e., shifts) depending on the stage. As shown in Figs. 14(a) to (c), in the scatter diagram 139 of the static matrix 131 for the super-healthy stage, there is almost no correlation, in the scatter diagram 139 of the static matrix 132 for the presymptomatic stage, a certain degree of correlation appears, and in the scatter diagram 139 of the static matrix 133 for the disease stage, high correlation is observed. In this way, it can be understood that the states of the scatter diagram 139 of the cells 138 in the matrices 13 of respective stages are changed (transformed) and the plots move depending on the stage.

This means that it is possible, when following test results are obtained at a second timing a certain time after the first timing when the previous test results 2 were obtained, to estimate transitions in the patient's condition by comparing (1) displacements in second relationships between a multitude of test items at the second timing, from the first relationships between the test items in the previous test results 2, and (2) the vector-appended transition matrices 14 for respective states. Accordingly, by comparing a state (second static state) estimated by comparing the second relationships for the current test and the static matrices 13 with a state estimated from the transition matrices 14, it is possible to verify a state that is estimated from the next test results and to estimate the state corresponding to the next test results with higher probability and accuracy.

Returning to Fig. 1, the examination system (the examination device, the examination apparatus) 10 includes: a first filtering unit (static filtering unit or static filter) 15 that uses the static matrices 13 for the respective stages in the library 12 as filters to generate test results 2 for the respective test timings for a patient who is the target complex system (individual complex system or first complex system) as individual matrices (first matrix and second matrix) 17 for the respective stages; and a second filtering unit (dynamic filtering unit or dynamic filter) 16 that generates displacements (transitions or differences) between the test results 2 with the transition matrices 14 indicating the transitions between stages as filters as individual displacement matrices (displacements or displacement matrices) 18. The examination system 10 also includes a first estimating unit (first estimation generator, first estimating engine, first estimation function or estimation means) 21 configured to estimate the health state (first state) of the patient (target system, subject) who is a first complex system based on the individual matrices 17 of each stage.

The examination system 10 is a system that estimates the state of an individual patient (target, subject), who is an individual first complex system, out of a plurality of patients (people) who are a multitude of target complex systems. As described earlier, the matrices (static matrices) 13 for respective stages include a large amount of correlation information for correlations between test results for a multitude of test items that suggest the state of a multitude of patients who correspond to the respective stages (super-healthy, presymptomatic, and diseased) in stages in changes over time that have been divided in a plurality of groups, out of the changes over time in the states of a multitude of patients who are the subjects to testing. In addition, each static matrix 13 includes a plurality of cells 138 that include correlation information for correlations between test results of a plurality of test items. The first filtering unit 15 converts test results for a multitude of test items for the subject (patient or first complex system) at the first timing into a matrix based on a large amount of correlation information on correlations between test results of a multitude of test items in at least one of the matrices out of the matrices 13 for each stage to generate an individual matrix (first matrix) 17. The first estimating unit 21 estimates, based on this individual matrix 17, whether the state of the patient at that time (the first state) is super-healthy or one of the presymptomatic levels, for example.

One method of generating an individual matrix 17 for the target is to reflect the relationships between the test results of a plurality of test items at the time of testing the target (that is, at the first timing) in each of the plurality of cells 138 included in the matrices 13 of each stage respectively. The first filtering unit 15 includes a first generating unit (first generator, first generating function or generating means) 15a that is configured to generate an individual matrix 17 according to this method.

Another method of generating an individual matrix 17 is to use a large amount of correlation information 136 between test results for a large number of test items in the matrices 13 for each stage to expand (map, lay out) the test results for a large number of test items at the time of testing the target (that is, the first timing) in each of the plurality of cells 138. The first filtering unit 15 includes a second generating unit (second generator, second generating function or generating means) 15b that is configured to generate an individual matrix 17 according to this method.

Fig. 16 depicts one example of an individual matrix 17 generated by the first generating unit 15. This individual matrix 17 indicates the relationship (correlation) between triglyceride (TG) and HRL cholesterol (HDL) as the relationship (first relationship) between the test items included in the individual matrix 17. As one example, the relationships between the items of three patients (targets, subjects) are plotted, and since the point of measured values (relationship) of patient A is at a position where the correlation is low (where there is low probability), this is indicated by the dot 171 that has a small area. On the other hand, since the point of measured values (relationship) of patient C is at a position with high correlation (high probability) that is close to the center 179 of the correlation, this is indicated by the dot 173 that has a large area. Since the point of measured values (relationship) of patient B is at a position with intermediate correlation (intermediate probability), this is indicated by the dot 172 that has a medium-sized area. As is shown, by using the static matrices 13 of the respective stages as filters, the test result of the first test of patient A for example is converted to a plurality of individual matrices 17 including images composed of a plurality of dots that have the same positions but different areas at each stage respectively. Even when the patient is different or the test results differ even for the same patient, by displaying with the static matrices 13 for respective stages as a filter, the positions within a large number of dots will differ and the areas of the respective dots for the target patient will also differ. Accordingly, for each test of each patient, the individual matrices 17 including different displays (outputs or images) at respective stages will be generated.

The first estimating unit 21, which is configured to estimate the health state (first state) of the patient (first complex system) using an individual matrix (first matrix) 17 includes an AI unit (first AI unit or state estimating AI) 20 configured to estimate the health state (first state) of the patient, who is a first complex system, from an individual matrix 17 of that patient using artificial intelligence produced by learning the correspondence between a plurality of individual matrices 17 and corresponding health states through machine learning. The AI unit 20 may be a system (apparatus) internally provided with artificial intelligence or may be a system for estimating a health state by using artificial intelligence provided outside the system 10. A typical AI unit 20 includes artificial intelligence that machine-learns, through image recognition on the individual matrices 17 including a plurality of cells 138, the correspondence between the plurality of individual matrices and the plurality of health states (first states) of the patients (targets).

As depicted in Fig. 16, the test results of each patient are expressed by a distance from the correlation information indicated in each cell 138 of the static matrices 13, for example, the positions of people exhibiting similar tendencies on a contour line 137. Accordingly, the test results of individual patients can be filtered and displayed by dots having different indices such as the sizes and/or colors (luminance) in the individual matrix 17 in proportion to the Euclidean distance from the center (the mean of two axes: a standard deviation of 50:50) of a certain stage, for example, presymptomatic level 3, where the number of patients is the highest. As one example, in a cell 138 in the individual matrix 17, the AI unit 20 can recognize the information of an individual patient as a dot of large size if the information is close to representative center and can recognize the information as a dot of a small size if the information is far from the center.

Although Fig. 16 has (two) XY axes and a representation using dots of two different sizes is given to enable identification as a pattern illustrating a characteristic recognized by AI, dots may be represented using colors or luminance, as described above. As another form of representation, as one example, it would be conceivable to handle the data as vector quantities (sizeTG, sizeHDL). In this case also, it is possible to use a configuration where the sizes of dots are changed in keeping with the Euclidean distance from the center, an emphasizing process is performed to indicate positions in the correlation more clearly, image recognition is performed by the AI unit 20 to grasp information indicated in the individual matrices 17, and as the result of machine learning, a health state corresponding to the individual matrix 17 is estimated. The method of reflecting the test results of a patient in an individual matrix 17 is not limited to this method, and another example will be described later.

The examination system 10 further includes a second estimating unit (second estimating engine, second estimation generator) 22 configured to estimate a transition in the state of the patient (the "first complex system") based on displacements between a next individual matrix (second matrix) and the individual matrix (first matrix) at the previous timing. The second matrix is produced by converting test results of a multitude of test items at a following test timing (second timing) of the patient (first complex system) when time has passed from the previous testing timing (first timing) to a matrix based on a large amount of correlation information for correlations between the test results of a multitude of test items in at least one of the matrices 13 for the respective stages.

The second filtering unit 16 includes a second individual displacement matrix generating unit (second individual displacement matrix generator) 16b that is configured to generate a displacement matrices 18 indicating differences between the individual matrices 17 generated at the first filtering unit 15 at respective test timings. The second filtering unit 16 further includes a first individual displacement matrix generating unit (first individual displacement matrix generator) 16a configured to generate a displacement matrix 18 where changes (displacements or transitions) in the test results of the individual matrices 17 (the first matrix and second matrix) at different test timings are added to the transition matrix 14 stored in advance in the storage 12.

Fig. 17 depicts examples of the individual displacement matrices 18 generated by the first individual displacement matrix generating unit 16a. Fig. 17(a) includes a displacement (transition or vector) 181 of the test result of patient A and a displacement (vector) 182 of the test result of patient B as examples, which are overlaid on the scatter diagram 139 for total cholesterol (TC) and low density lipoprotein (LDL) for diseased. Fig. 17(b) depicts an individual displacement matrix 18 visualized by using the vector-appended scatter diagram (vector map) 143 of the transition matrix 14 depicted in Fig. 13 as a filter, which are overlaid on the scatter diagrams 139 for total cholesterol and low density lipoprotein (LDL) for diseased. Since the vector 181 of patient A is located in a low-density part (indicating rare cases) of the vector map 143, the vector 181 is not emphasized or is reduced. On the other hand, since the vector 182 of patient B is located in a high-density part of the vector map 143, the vector 182 is emphasized.

As described above, the displacements of the test results of patient A are converted, using the vector-appended transition matrices 14 of respective states as a filter, into a plurality of individual displacement matrices including images composed of a large number of vectors with common positions but different vector magnitudes. When there are no displacements, the vectors are displayed as dots or are not displayed. If the displacements in the test results differ for different patients or even for the same patient, the positions will differ for a large number of vectors or dots, and the sizes of the respective vectors will also differ. Accordingly, by using the displacements in the test results for each patient, individual displacement matrices 18 with different images in each state are generated.

The second estimating unit 22 includes a second AI unit 20 configured to estimate transitions in the health state of the patient using artificial intelligence produced by learning the correspondence between displacements between the plurality of first matrices 17 and the plurality of second matrices 17 and changes in state over time of a multitude of patients who are target complex systems using machine learning. In the present embodiment, the AI unit 20 is shared with the AI unit described above. The AI unit 20 compares the images included in the individual displacement matrix 18 with similar images of a large number of patients from the past to estimate a state corresponding to the displacements for the test results of the patient and is capable of verifying the latest health state of the patient statically estimated by the first estimating unit 21. The AI unit 20 has a function which performs image recognition, when a patient has different test results 2 over time and the individual displacement matrices 18 of respective states are obtained, on the individual displacement matrices 18 and verifies the state of the patient estimated using the individual matrices 17.

As described above, the examination system 10 includes a static determination unit (the first estimating unit) 21 that compares first relationships between a multitude of test items at a first timing (the previous time, the present time, or the next time) of a human (patient) who is a target complex system and a multitude of correlations between a multitude of test items in the static matrices 13 and estimates the health state (a first static state) of the patient at each of those timings. The examination system 10 also includes a dynamic determination unit (second estimating unit) 22 that compares displacements in second relationships between a multitude of test items at a second timing (the present time or next time) for the patient when time has passed from the first timing, with respect to the first relationships and transition matrices 14, for example vector maps, from each stage, and verifies the second static state estimated each time by the static determination unit 21 based on the second relationships.

In the present embodiment, these units 21 and 22 include a shared artificial intelligence unit (AI unit) 20, and the AI unit 20 may also include a function 23 for performing image recognition on the relationships between the first relationships and the static matrices 13 at each stage and estimating the first static state at each time. In one example, the AI unit 20 may include a function that estimates the first static state at any timing by performing image recognition on the individual matrices 17 at respective states which were produced by converting the first relationships that are static relationships between the test items into images, with the static matrices 13, for example, with the static matrices 13 appended with contour lines as filters.

The AI unit 20 may further include a function 24 that performs image recognition on the relationships between the displacements between test results and the transition matrices 14 for each stage and estimates a second static state of the patient for when testing is performed after further time has passed. In other words, this function (unit) 24 is configured to compare the displacements in the individual matrices (the first matrix and second matrix) 17 at different test timings with the transition matrices 14 to verify the latest estimated health state of the patient based on the individual matrix (second matrix) 17 at the present time. As one example, as described above, the AI unit 20 may also include a function that performs image recognition on the individual displacement matrix 18 for each state produced by converting the displacements between the test results into images using the transition matrices 14 for respective stages as a filter and estimates and confirms a second health state of the patient for a predetermined time later that was statically estimated.

The AI unit 20 may further include a unit 25 configured to estimate the future transitions (transitions after time) for the patient from present, next (second timing), or subsequent state. From filtered results, it is possible to predictively estimate a position (stage) of the present health state and transition risks for the health state (symptoms) in the future. That is, displacements (transitions) in the state of the patient up to now are emphasized by vectors in the filtered individual displacement matrix 18, and by using images including a large number of vectors obtained from the test values out of a large number of test items, the AI unit 20 is capable of comparing with similar images of a large number of patients in the past to estimate future state transitions for the patient.

The AI unit 20 can estimate the states corresponding to the test results of patients by performing image recognition processing on the images included in individual matrices 17. That is, the AI unit 20 can estimate the present state of a patient by comparing with similar images of a large number of patients from the past. Accordingly, the AI unit 20 can estimate the next transition destinations by starting from the present states of the patients. In addition, when the individual matrices 17 and/or the individual displacement matrices 18 of the patients are completely outside representative regions, or the probabilities are low, it is possible to additionally perform various kinds of processing, such as trying to match with static matrices 13 or transition matrices 14 of other stages, repeating the determinations, and creating a new classifications, stages, and/or transition paths, as an exception processing. This exception processing is then sequentially registered as new matrices, stages, or paths and handled by the AI unit 20 as new knowledge so that even complex cases are subsequently recognized and estimated without being treated as exceptions.

The examination system 10 may further include an output unit (output device, interface) 35 configured to output one or more individual matrices (first matrices) 17 where a plurality of cells 138 that have a large number of test items as X and Y axes are arranged in two dimensions. Although the cells 138 and the respective types of matrices are depicted in two dimensions in the figures described above, the cells and the matrices processed by the examination system 10 and the AI unit 20 do not need to be two dimensional and may be three dimensional or multi-dimensional. On the other hand, it is necessary for an specialist, such as a doctor or a technician, who reviews the estimates (examination results, diagnosis results) produced by the examination system 10 and the AI unit 20 to understand the factors that led the examination system 10 and the AI unit 20 to produce those results. For this reason, it is useful to output the cells and matrices in a state in which they can be easily understood by humans.

The examination system 10 may further include a unit (generator) 30 that automatically generates the static matrices 13 and the transition matrices 14 from statistical data. When a health state (symptoms) that has been estimated corresponding to the test results 2 of a patient has been established with high accuracy and confirmed by a doctor who is a specialist, the automatic generating unit 30 adds such test results 2 that have been confirmed to the static matrix 13 for that stage. At the same time, it is determined whether a new trend has been observed for each correlation between the test items, and when a new trend has been determined, replicas (pseudo data/replicated data) are added to that actual data to greatly increase the number of samples to be included in those static matrices 13 and in that transition matrices 14. By increasing the number of samples in the static matrices 13 and the transition matrices 14, it is possible to automatically generate individual matrices 17 and individual displacement matrices 18 corresponding to the number of samples, which further promotes learning by the AI unit 20.

Fig. 18 depicts how the automatic generating unit 30 increases the number of samples included in a static matrix 13 using replicas. In the scatter diagram 139 indicating the correlation between triglyceride (TG) and HRL cholesterol (HDL) in the static matrix 132 for presymptomatic, when a new correlation is found in Fig. 18(a), a replica model including the new correlation is generated as depicted in Fig. 18(b), and samples corresponding to the new correlation are generated so as to form a normal distribution. If a plurality of normal distributions are included, the number of samples can be increased by mixing at 50:50 or another ratio, so that this process can increase the number of samples by an order of magnitude or more, for example.

Fig. 19 depicts a different example of individual matrices 17. The shown individual matrix 17 is an example of a matrix generated by the second generating unit 15b, where test results of a multitude of test items at the time of testing of the patient have been expanded into (mapped or laid out on) a plurality of cells 138 according to predetermined rules using the correlation information 136 in the static matrices 13 to which the patient belongs. Although BMI (body mass index), LF/HF (heart rate variability, sympathetic nerve, parasympathetic nerve ratio), BP/Land BP/H (blood pressure), Heart R (heart rate), HbAlc (hemoglobin A1c), TP (total protein), Alb (albumin), AST (aspartate aminotransferase), ALT (alanine aminotransferase), γ-GTP (γ glutamyl transpeptidase), BUN (blood urea nitrogen), eGFR (estimated glomerular filtration rate), AMY (amylase), UA (uric acid) and LDL (LDL cholesterol) are given here as examples of the test items, the present invention is not limited to these examples.

The individual matrix 17 depicted in Fig. 19 is a heat map 175 in which these test items are arranged on the X and Y axes. The results of these test items are displayed in the diagonal cells 138d and when the test results are average values, the corresponding cells 138 are indicated in white. If the test result is lower than the average value (i.e., favorable), this is indicated using 10 levels of shadings of the color blue according to the deviation value. If the test result is higher than the average value (i.e., unfavorable), this is indicated using 10 levels of shadings of the color red according to the deviation value. In addition, in accordance with predetermined rules, in order for the test results for the respective test items to be reflected in the correlation information (correlation coefficients) 136 of the test results for correlations between the test items included in the static matrices 13, a shift is performed in the favorable direction or unfavorable direction and the color or shade is changed to make it easier to express the health state of the patient as a pattern in a heat map. Fig. 19(a) depicts a heat map 175 displayed using colors and shades in a gray scale and Fig. 19(b) depicts a heat map 175a where the shade of each color has been replaced with the symbols depicted in Fig. 19(c). The same applies to the following figures.

This heat map 175 is an individual matrix 17 converted to two dimensions and is one example of a favorable representation for use in image analysis. It is possible to evaluate the test results of individual test items not only as test results for such test items but also in association using correlation coefficients for other test items (including measurements, medical interviews, etc.) and thereby evaluate the health state of the patient. In addition, the health state can be evaluated by considering the correlations at each stage which are gathered into the static matrices 13 of the respective stages such as super-healthy, the presymptomatic levels, and diseased. It is also possible to evaluate the health state with consideration to correlations (group correlations) between a plurality of test items. As one example, glucose, HbA1c, and fructosamine are known to have a group correlation with pancreatic disease. In the static matrices 13, since the respective correlation coefficients for such groups are highly reflected, if a test result of one test item belonging to a group exhibits a poor value (a deviation), in many cases the test results of other test items belonging to the same group will also exhibit poor values. Using such characteristics of the static matrices of respective stages, in the heat map 175, for test items whose group includes the test items on the X-axis and the Y-axis, the value of a cell where the test items intersect is displayed having been shifted in the unfavorable direction. This means that the test results of the test items belonging to the group are highlighted in the unfavorable (poor) direction.

On the other hand, when the test items belonging to the group are favorable, the test items are shifted in the direction of improvement. For test items that have hardly any correlation, the test results are displayed as they are without amendment. Depending on the state of the patient at the time of testing, there are also cases where the test results of the test items that are expected to be correlated with each other do not exhibit correlation, and there are cases where the test results exhibit inverse correlation. Although various states may occur depending on the patient, by using static matrices 13 produced by statistical processing that divides a large number of patients into stages, it is possible to estimate and make determinations on the state of health of a patient at various timings more accurately using the image recognition ability of artificial intelligence.

The method (that is, the rules and shifting method) of generating the heat maps 175 used in the present embodiment will now be described with reference to Fig. 20. The rules are set for four cases according to the deviation value Xj of the test item (input item) on the horizontal axis (X axis) of the heat map 175 and the deviation value Yj of the test item on the vertical axis (Y axis).

1. When 50<Xi and 50<Yj

In this case, since the test results of both test items are on the healthy side (their colors are white to blue), the Z scores ZSxi and ZSyj for the test items on the X and Y axes are used, and when the deviation value Xi of the test item on the X-axis is relatively large, a proportionally larger shift $\Delta s$ is generated and a deviation value indicating the displaying of the cell 138 at the intersection with the test item on the Y-axis direction is generated. In addition, the following conditions are included.

- When the deviation value Xi is around 50 to 60, a shift $\Delta s$ is hardly produced.

- When the deviation value Xi is 50, the deviation value Yj of the test item on the Y axis is maintained without being shifted.

- When the correlation coefficient CCij included in the correlation information 136 of each cell 138 in the static matrices 13 is larger than 0.2, the healthy tendency or deterioration tendency is further strengthened in keeping with the coefficient.

- When the correlation coefficient CCij is smaller than -0.2, the deviation value Yj of the test item on the Y axis is conversely weakened.

- When the correlation coefficient CCij is outside the ranges indicated above (including zero), the same deviation value Yj is maintained without shifting.

One example of a conversion formula for calculating the deviation value SCij for displaying the cell Cij so as to satisfy above conditions is as follows.
When CCij is positive (and CCij>0.2)

$$SCij = (\Delta s + 1.0) \times Yj$$

$$\Delta s = k1 \times (\sqrt{(|(ZSyj + 1.0) \times (ZSxi + 1.0) \times CCij|)} - 1.0) \times k2 \times k3$$

where Z score ZS is as follows.

$$ZS = (xi - \mu x) / \sigma x$$

where xi is a sample value, $\mu$x is the arithmetical mean, and ox is the standard deviation.

The constant k1 is a proportionality constant, as one example, 0.6. The constant k2 is the shift ratio, and the constant k3 is a weight factor. The same applies to the following description.

When CCij is negative (and CCij<-0.2)

$$SCij=(\Delta s+1.0)\times Yj-50\times\Delta s$$

$$\Delta s=k1\times(\sqrt{(|(ZSyj+1.0)/(ZSxi+1.0)\times CCij|)}-1.0)\times k2\times k3$$

2. When Xi<50 and 50<Yj

In this case, the deviation value Xi of the test value of the test item on the X axis is less than 50, so that a tendency toward the diseased state is suspected. Since the deviation value Yj of the test value of the test item on the Y axis indicates healthy (white to blue), the shift amount in the case of where correlation coefficient CCij is positive is a shift from the actual value toward an average value (white), that is, a shift that changes from the original blue color to a light color. When the correlation coefficient CCij is negative, the shift is in the opposite direction. One example of a conversion formula for calculating the deviation value SCij for displaying the cell Cij in this case is as follows.

When CCij is positive (and CCij>0.2)

$$SCij=(\Delta s+1.0)\times Yj-50\times\Delta s$$

$$\Delta s=k1\times(\sqrt{(|(ZSyj+1.0)/(ZSxi-1.0)\times CCij|)}-1.0)\times k2\times k3$$

When CCij is negative (and CCij<-0.2)

$$SCij=(\Delta s+1.0)\times Yj$$

$$\Delta s=k1\times(\sqrt{(|(ZSyj+1.0)\times(ZSxi-1.0)\times CCij|)}-1.0)\times k2\times k3$$

3. When Xi<50 and Yj<50

In this case, the deviation value Xi of the test value of the test item on the X axis is less than 50, so that a tendency toward the diseased state is suspected. In addition, the deviation value Yj of the test result of the test item on the Y axis to be compared is also less than 50, which suggests a tendency toward a diseased state in the same way. Accordingly, there is a tendency toward the diseased state for both test items, and when the correlation coefficient CCij is greater than 0.2, the position is shifted to the red side, which emphasizes the diseased state in keeping with the coefficient. When the correlation coefficient CCij is smaller than -0.2, conversely, an operation that weakens the red color indicating the tendency toward diseased is performed. When the correlation coefficient CCij is between those ranges (including zero), the deviation value Yj of the test result of the test item on the Y axis is maintained without shifting. One example of a conversion formula for calculating the deviation value SCij for displaying the cell Cij in this case is as follows.

When CCij is positive (and CCij>0.2)

$$SCij=(\Delta s+1.0)\times Yj-50\times\Delta s$$

$$\Delta s=k1\times(\sqrt{(|(ZSyj-1.0)/(ZSxi-1.0)\times CCij|)}-1.0)\times k2\times k3$$

When CCij is negative (and CCij<-0.2)

$$SCij=(\Delta s+1.0)\times Yj$$

$$\Delta s=k1\times(\sqrt{(|(ZSyj-1.0)\times(ZSxi-1.0)\times CCij|)}-1.0)\times k2\times k3$$

4. When 50<Xi and Yj<50

In this case, the deviation value Xi of the test result of the test item on the X-axis is on the healthy side, but the deviation value Yj of the test result of the test item on the Y-axis is suspected to have a tendency toward diseased. For this reason, when the correlation coefficient CCij is larger than 0.2, a process for further weakening the tendency toward diseased is performed in accordance with the coefficient, and conversely when the correlation coefficient CCij is smaller than -0.2, a process that strengthens the tendency toward diseased is performed. An example of the conversion formula for obtaining the deviation value SCij for displaying the cell Cij in this case is as follows.
When CCij is positive (and CCij>0.2)

$$SCij=(\Delta s+1.0)\times Yj-50\times \Delta s$$

$$\Delta s=k1\times(\sqrt{(|(ZSyj-1.0)/(ZSxi+1.0)\times CCij|)}-1.0)\times k2\times k3$$

When CCij is negative (and CCij<-0.2)

$$SCij=(\Delta s+1.0)\times Yj$$

$$\Delta s=k1\times(\sqrt{(|(ZSyj-1.0)\times(ZSxi+1.0)\times CCij|)}-1.0)\times k2\times k3$$

As one example, in Fig. 20, it is assumed that the deviation value of the test result for BMI is a favorable value of 50 or more, as one example, around 60, the deviation value of the test result for HbAlc is a value of 50 or less, for example, 40 or less, and the test result for eGFR is 50 or less, as one example, 45 or less. As depicted in Fig. 20(c), it is assumed that weak positive correlation has been identified between BMI and eGFR, and strong positive correlation has been identified between BMI and HbA1c. In this case, as depicted in Fig. 20(b), the BMI test results in the cells 138x and 138y indicating the relationships with eGFR and HbAlc are displayed with a less deep shade of blue, which indicates favorable. For the test results of eGFR and HbAlc in cells 138v and 138u indicating the relationship with BMI are displayed in the red tint, which indicates unfavorable weakly.

The shift ratio k2 may be 1, and the shift amount may be corrected at a stage where the group correlation (a tendency to diseased or the healthy triangle) becomes visible. The shift amount may be changed according to conditions such as the early stage of a tendency to diseased, the immune system having been weakened, and other conditions for the patient, such as sleep, diet, stress, or physical exhaustion. By changing the weight factor k3 with consideration to the influence of a specified disease, a certain drug, and the living environment, it is possible to generate heat maps 175 that are appropriate for displaying specific changes with greater focus. When using the AI unit 20 to perform further analysis focusing on these conditions, the weight factor k3 can be made to function as an adjustment parameter.

Although the processing method described above is an example of a processing method where shift amounts Δs are used to emphasize differences in shades of colors in the heat maps 175 depending on the positions (meanings) of the deviation values so that the patient's state can be seen more clearly, the method of generating such heat maps 175 is not limited to this. However, the processing method described above is based on simple multiplication and division and uses the Z scores indicating the position of the deviation value, and is therefore one of preferred methods in that a pattern that is easily recognized as an image is formed without performing very complicated processing. In particular, it is possible to emphasize that the meaning of the deviation value of the test result changes significantly at the average of 50, and if the deviation value becomes large, it is necessary be consider the result as a case that probabilistically rarely occurs, which can also be reflected in the pattern.

Fig. 21 depicts an example of a heat map (individual matrix) 175 of the same patient at a time (second timing) several months later. By comparing with the heat map in Fig. 19, it is possible to easily grasp the change in the patient's state over several months as the differences in the pattern. Accordingly, the AI unit 20 can estimate the state of the

16

patient at each time by recognizing the pattern of the heat map 175, and can also estimate transitions in the state of health of the patient.

Fig. 22 depicts an example of displacement matrices 18 generated by the second individual displacement matrix generating unit 16b. The displacement matrix 18 depicted in Fig. 22 is a heat map (differential heat map) 185 obtained by extracting the differences in color (blue and red) and the differences in levels of shade between the heat map 175 depicted in Fig. 19 and the heat map 175 depicted in Fig. 21. In the AI unit 20, by performing image recognition on the pattern appearing in the differential heat map 185 and comparing against the results of machine learning, it is possible to more clearly estimate transitions in the state of health of the patient. In Fig. 22, Fig. 22(a) depicts the differential heat map 185 using shades of colors and Fig. 22(b) depicts a differential heat map 185a in which the shades of the respective colors have been replaced by the symbols in the drawing.

Fig. 23 depicts an overview of the processing in the examination system 10. This processing 500 is a method for using a computer 50 to estimate the state of individual complex systems out of a multitude of target complex systems. In step 510, the first filtering unit 15 determines a stage to which a patient belongs (that is, the stage to which the state of health of the patient belongs) in order to extract the static matrix 13 to be used for filtering. The stages to which the patient's state of health belongs changes over time and can be determined based on the deviation values of the test results of all or some of a large number of test items, for example. The test results for each test item appear in the cells 138d arranged diagonally in the heat map 175 depicted in Fig. 19. It is therefore possible to determine the stage of health to which the patient belongs and to select the static matrix 13 for that stage based on the pattern of the colors and shades of the diagonal cells 138d. The selected static matrix 13 is not limited to one, and static matrices 13 of a plurality of stages that may correspond to the state of health of the patient may be extracted.

In step 520, the first filtering unit 15 generates an individual matrix 17 for the patient. In step 520, the individual matrix (first matrix) 17 is generated by converting the test results of a multitude of test items at the first timing for the patient who is an individual complex system to a matrix based on a large amount of correlation information 316 for correlations between the test results of the multitude of test items in one or more selected static matrices 13. In this step, it is possible to generate one or more individual matrices 17 by reflecting the relationship between test results of a plurality of test items at the first timing for the patient, who is a first complex system, on each of the plurality of cells 138 included in the matrices 13 of respective stages. It is also possible to generate one or more heat maps 175 by expanding or mapping the test results of a large number of test items at the first timing of the patient to a plurality of cells 138 using a large amount of correlation information 316 for correlations between the test results of a large number of test items in the matrices 13 of each stage.

In step 530, the first estimating unit 21 estimates the state (state of health) of the patient at this time based on one or more individual matrices 17. In this step 530, it is possible to estimate the state of health of the patient using the AI unit 20 which includes artificial intelligence produced by learning the correspondence between a plurality of individual matrices 17 and a plurality of health states have been learned through machine learning.

In step 540, the second filtering unit 16 generates the individual displacement matrix 18 that includes displacement information of displacements (transitions) between (1) an individual matrix 17 in which the test results of test items for the patient at the second timing where time has passed from the first timing are collected and (2) the individual matrix 17 for the first timing. In this step 540, one or more individual displacement matrices 18, where displacements in the patient's state of health have been reflected in the transition matrices 14, and/or a differential heat maps 185 may be generated.

In step 550, the second estimating unit 22 estimates the transitions in the state of health of the patient based on one or more displacement matrices 18. Step 550 may include a process of verifying whether the state of the patient indicated in the newest individual matrix 17 matches a state estimated from one or more past individual matrices 17. Step 550 may also include a process of estimating future transitions in the state of health of the patient. In step 560, the examination system 10 outputs a diagnosis result 3 including the estimated state of health of the patient, outputs information, such as a heat map, as the basis for this diagnosis result, and the estimated state of health is then confirmed by a doctor who is a specialist. Based on this estimated state of health, the doctor may add more test items or set a treatment policy as necessary.

A program (program product or software) 51 for causing the computer 50 to execute these processes can be provided having been recorded on an appropriate recording medium, or can be provided via a computer network such as the Internet.

The examination system (diagnostic system) 10 described above is not limited to health care and illnesses, and can estimate the state of any complex and sophisticated system based on regular or continuous observation data. This means that the present invention can be applied to a system that performs automatic replacement, repair, and/or maintenance due to deterioration in the activity of the system as a whole or in individual operation states, or deterioration, failure, or abnormalities (including changes over time) of component parts. With this system 10, a system including artificial intelligence (AI) can predictively estimate normal values and failure/abnormality risks for an advanced system from the correlations between fluctuating values of a plurality of parameters.

One example of the systems that include AI is a hybrid system (diagnostic system) including two engine systems, that is, knowledge-based inference engines (estimating engine, including a verification engine and an optimization engine) and a deep-learning AI engine. Such hybrid system is one example of the systems that are capable of predictively estimating the state of health and disease risk (that is, normal values and failure/abnormality risks for an advanced system).

Fig. 24 depicts another example of a diagnostic system. For this diagnostic system 300, the target (subject) of diagnosis is people (humans, the human body). This diagnostic system (Axion system) 300 includes a hybrid engine (diagnosis engine or Axion engine, or AXiR engine) 320 which includes: artificial intelligence (AI) 321; an inference engine (estimating engine) 322 with a function as an expert system of doctors who examine (make diagnoses for) humans as the targets; and a teaching system 330 that generates input replicas 313 including replicas of the input data for teaching the artificial intelligence 321 about diagnosis results of the artificial intelligence 321 and the inference engine 322 for the input data 311. The input data 311 is provided to the inference engine 322 and the AI engine 321 via an input processing module 325, a pre-processing module 326 and a correlation processing module 327.

The diagnosis engine 320 includes a verification engine 323 that verifies the outputs of the artificial intelligence (AI engine) 321 and the inference engine 322, an optimization engine 324 that optimizes the input replicas 313 based on the verification results of the verification engine 323, and a replica generation module (replica generator) 319 that generates input replicas 313 in accordance with the optimization requirements of the optimization engine 323. Although the replica generator 319 is configured using separate hardware to the diagnosis engine 320 in the hardware configuration in this example, these elements may instead be configured of the same hardware.

In this hybrid-type diagnosis engine 320, first, the AI engine 321 is trained using input replicas 313 generated by receiving feedback from the verification engine 323 and the optimization engine 324 to realize automatic or autonomous improvements in diagnostic accuracy. Next, since the diagnosis engine 320 is a hybrid equipped with both the inference engine 322 and the AI engine 321, after the AI engine 321 has been trained, it is possible to make inferences and estimates, from input data 311 including non-invasive and/or invasive measurement data, about the activity state (healthy/unhealthy/presymptomatic), dysfunctions/deficiencies (the extent of disease), and deterioration/tumors/carcinogenesis for internal elements of the human body from the perspectives of both the inference engine 322, which is an medical expert system, and the AI engine 321, and to provide advice, health information that is of reference, and viewpoints (potential risks and dangers) that are likely to be overlooked.

In other words, the diagnosis engine 320 is characterized by the self-learning function of the AI engine 321 and this self-learning function being supported by the functioning of two engines, that is, the verification engine 323 and the optimization engine 324 with the aim of improving the accuracy of predictions and inference. In particular, although the inference engine 322 and the AI engine 321 function as a hybrid, the system is also characterized in that switching is performed when the prediction/inference precision of the AI engine 321 continuously exceeds that of the inference engine 322. The replica generation module 319 is externally provided so that it can function even on a small amount of input data, and is constructed so that the variations used in replica generation change according to instructions from the optimization engine 324.

The AI engine 321 implemented here is based on deep learning 350. To enable the AI engine 321 to function on even a little input data 311 (to promote autonomous learning), the teaching system 330 uses three engines, the inference engine 322, the verification engine 323, and the optimization engine 324 to support the AI engine 321 and play a partial role in the self-learning function so as to support the growth of the AI engine 321. The three engines 322 to 324 of the teaching system 330 also have a function of improving the prediction accuracy and inference accuracy of the diagnostic system 300 and the AI engine 321. That is, the teaching system 330 functions as a type of feedback circuit for improving accuracy.

Thanks to the teaching system 330, the AI engine 321 will gradually exceed the prediction accuracy of the inference engine 322. The win ratio between the AI engine 321 and the inference engine 322 is determined by the verification engine 323 and a final determination/decision module 328 that makes a final determination further downstream. At an initial stage, the engine 323 and the module 328 give priority to the diagnosis result (inference or prediction) of the inference engine 322. After this, as examples, when the win ratio of the AI engine 321 exceeds 80% on average for three months or the win ratio continuously exceeds 90%, the verification engine 323 and the final determination/decision module 328 give priority to the diagnosis result of the AI engine 321 and the inference engine 322 is placed on the backup side so that its inferences and predictions are not used for the time being. However, the verification engine 323 and the optimization engine 324 continue to function and give support even when the AI engine 321 is operating as the main engine.

Accordingly, the functioning, nature, performance, and the like of the diagnosis engine 320 are influenced by the three engines included in the teaching system 330, that is, the inference engine 322, the verification engine 323, and the optimization engine 324. These engines 322 to 324 are fundamentally based on the concept of a meta model. In other words, in the system to be diagnosed, a complex system such as the human body for which it is necessary to predict abnormalities and failures with high accuracy, there are system elements and components that construct the system, and the functioning of these elements or components serves as the basis for predictions and enable the inputs and outputs of the system to be defined. Here, it is possible to assume that a favorable, unfavorable, defective, or broken down state of the target systems of diagnosis has occurred from a problem with internal components or parts, regardless of the extent of the state, and use a design that predicts the causes or problems from measurement data and/or observation data, gives advice on countermeasures, and provides information that is necessary and/or information that is expected for that situation .

This configuration uses meta model description files 314, in which the functions of these engines 322, 323, and 324 are written, to model a set of definitions and/or correlation information, input information, statistical information, and evaluation information to the greatest extent possible on the outside, so that the configuration can be adapted to a plurality of systems and applications by changing the written content of the description information. Accordingly, by changing the input data 311 and the meta model description file 314, the diagnosis engine 320 can have a different role and range of protection/response. In addition, even in the same target field of application, by using specialized input data 311, it is possible to create (train) diagnosis engines 320 that have different specialties and personalities. One example is a system with a premise of a meta model description file 314 based on input data 311 in different fields, such as health measurements, breast cancer, pancreatic cancer, kidney cancer, lung cancer, stomach cancer, liver cancer, colon cancer, and the like in the health care field. However, the field in which the diagnosis engine 320 can be applied is not limited to health care and may be an industrial field, such as gas turbine engines and factory automation, a commercial or service field such as finance, or an entertainment field, such as (video) games as described earlier.

The inference engine 322 is defined by the meta model description file 314 and indicates correlations using the input data 311 that is a basis for estimations about internal parts that are in a direct relationship or a causal relationship with a favorable, unfavorable, disordered, failing, or damaged state of the target complex system subject to diagnosing, and about their relationships, activity levels, recovery function, immune system, adjustment mechanism, and deterioration factors. The inference engine 322 estimates internal replicas 313 for the target system from the input data 311 and sets this as a first-order prediction. If this first-order prediction is reliable within a certain range, the input data is re-estimated as a second-order prediction. From this second-order prediction, the timing and damage of the favorable, unfavorable, disordered, failing, or damaged (boundary) state of the system that was previously of concern is estimated as a third-order prediction.

When determining the output data 312 via an output processing module 329, data out of the input data (which may include measurement data, a medical questionnaire, or the like) 311, the replicas 313 (that is, internal replicas) that are generated from the input data 311, and also regenerated input replicas that are regenerated from a variation of the internal replicas which has the smallest difference (variance) with the measurement data may be given as leading candidates for the inference result. Here, the correlation with the input data 311 is estimated from the description file. Since the verification engine 323 is available, when narrowing down is difficult, the estimation accuracy may be improved by performing a supplementary test and obtaining new input data 311 and medical interview data. This is also used by the optimization engine 324 and contributes to improvements in prediction/estimation accuracy. It is also necessary to regularly review and update the input/output correlation table/description file 315 to ensure these improvements in accuracy. It is necessary for the correlation table information to include information indicating the relationship between input data and/or medical interview information and internal parts of the human body (organs, blood vessels, bones, the spinal cord, and the like). The output data 312 may be outputted via the PE engine 318 that performs social-type analysis of the patient.

Input replicas 313 are data that are generated from one or more probability models based on statistical data and/or real data, and are used in place of actual measurement data. For a probability model specified from mean values and variance, the input data 311 for health care includes a blood test, a urine test, breath/skin gas measurements, measured values of body temperature, visceral fat, subcutaneous fat, muscle mass, body fat percentage, estimated bone mass, blood pressure, blood flow, heart rate, autonomic excitability and the like, image diagnosis results, microRNA, ncRNA, DNA or the like, and medical questionnaires.

The input replica generating module 319 is a module that generates data (the "input replicas") 313 to be used in place of actual measurement data based on statistical data and real data. The replica model may be a non-correlation model (also referred to as a "simple model", a model where there is no correlation between data), and as described earlier, may be a model having correlations (correlation model or hierarchical model) with super-healthy, presymptomatic, or the like. A correlation model can be introduced via the input/output correlation table 315. The correlation table 315 is a file that defines correlations between the input data (test data) 311 and indices of internal states (such as the activity rates of respective parts). Accordingly, for an application where a normal range and an abnormal range are statistically known for the measurement data, the correlation processing module 327 is capable of defining internal states relating to these ranges as indices for a statistical model including the static matrices 13 and the transition matrices 14 described earlier. While learning a plurality of correlations (quantities) and patterns of diseases as disease risks, the AI engine 321 can learn shift paths as a disease tree or disease map. By further including other standard variables related to maintaining health, it becomes possible to determine the paths of shifts to diseased. The system 300 including the diagnosis engine 320 can therefore function as the diagnostic system 10 described earlier.

The above description discloses a system 10 for estimating the state of a first system subject to testing out of a large number of (a multitude of) target systems (complex systems). This system (examination system or diagnostic system) includes: a first unit 11 that stores, as static matrices 13 for respective states that are collections of (a multitude of) correlations between a large number of (a multitude of) test items in each state for a multitude of target systems, and each state is out of a plurality of defined static states out of a plurality of states of the target systems that change over time; and a static inference engine (static estimating engine, static determination unit) 21 that compares first relationships between a multitude of test items at first timing of the first system subject to testing and the multitude of correlations between the multitude of test items in the static matrices for respective states and estimates a first static state of the first system.

The static inference engine 21 may include a first AI unit (artificial intelligence unit) 20 that estimates the first static state by performing image recognition on the relationship between the first relationships and the relationships in the static matrices for the respective states. The first AI unit 20 may include a unit that estimates the first static state by performing image recognition on individual matrices for each state produced by converting the first relationships to images with the static matrices 13 for the respective states as filters.

The examination system 10 may further include: a second unit 12 that stores dynamic matrices 14 for respective states, which include displacements or transitions of a multitude of correlations between the static matrices of the respective states and reflect changes in state over time of a multitude of target systems; and a dynamic inference engine (dynamic estimating engine, dynamic determination unit) 22 that compares displacements, with respect to the first relationships, of second relationships between a multitude of test items of the target system at second timing where time has passed from the first timing, and the dynamic matrices of the respective states and verifies a second static state estimated by the static inference engine based on the second relationships. The dynamic matrices may include displacements of the multitude of correlations from other states and may include displacements of the multitude of correlations to other states. In addition to the static correlations between a multitude of test items, it is possible to compare changes over time in a multitude of test items with the dynamic matrices of the respective states, which makes it possible to provide more accurate estimates of symptoms.

In addition, the dynamic inference engine 22 may include a function of estimating transitions in the state of the first system subject to testing from the second timing onward. Since the dynamic inference engine estimates the state based on past progressions, it is possible to estimate future progressions by extending the past progressions. The dynamic inference engine 22 may include a second AI unit (in the present embodiment, a shared AI unit) 20 that performs image recognition on the relationships between the displacements and the dynamic matrices of each state and estimates the second static state. This second AI unit may be shared with or independent of the first AI unit. Also, the second AI unit may include a unit that performs image recognition on the individual displacement matrices of the respective states obtained by converting the displacements into an image with the dynamic matrix of each state as a filter, and estimates the second static state. The system 10 may include an automatic generating unit 30 that adds at least one of first relationships and second relationships to the static matrices of respective states and the dynamic matrices of respective states based on verification results of the dynamic inference engine. The automatic generating unit may include a unit that automatically generates a multitude of correlations between a multitude of test items in each state using replicas of the target systems.

Another aspect disclosed above is a method of estimating the state of a first target system out of a large number of target systems using AI (artificial intelligence). The AI may be capable of accessing a first unit that stores a large number of correlations between a large number of test items in respective states for a large number of the target systems for respective status of a plurality of defined static states out of a plurality of states of the large number of target systems that change over time, as static matrices of respective states that are collections of the large number of correlations. And the method may include a first step that compares first relationships between a large number of test items at first timing of a first target system and the large number of correlations between the large number of test items in the static matrices for respective states and estimates a first static state of the first target system. The first step may include estimating the first static state by performing image recognition on individual matrices for each state produced by converting the first relationships to images with the static matrices for the respective states as filters.

The AI may be capable of accessing a second unit that stores dynamic matrices for respective states, and the method may include a second step that compares displacements, with respect to the first relationships, of second relationships between the large number of test items of the target systems at second timing when time has passed from the first timing, and the dynamic matrices of the respective states, and verifies a second static state estimated by the static inference engine based on the second relationships. The second step may include estimating the second static state by performing image recognition on an individual displacement matrix of each state produced by converting the displacements of the second relationships with respect to the first relationships to an image using the dynamic matrices of the respective states as a filter. The method may further include a third step of estimating transitions in the state of the first target system from the second timing onward.

Note that although an examination system where the human body (humans) is the target complex systems has been described above, the target complex systems may be animals, including livestock and pets, or plants, hardware (mechatronics) such as manufacturing or producing plants, ships, vehicles, or engines, or may be software such as applications or AIs.

**Claims**

1. A system that estimates a state of an individual complex system out of a multitude of target complex systems, comprising:

   first storage that stores matrices of respective stages that include a large amount of correlation information for correlations between test results of a multitude of test items that suggest states of the multitude of target complex systems corresponding to stages in changes over time of the multitude of target complex systems, the matrices including a plurality of cells including correlation information for correlations between test results of a plurality of test items; and
   a first estimating unit configured to estimate a first state of a first complex system that is an individual complex system based on a first matrix produced by converting test results for the multitude of test items at first timing of the first complex system into a matrix based on the large amount of correlation information for correlations between the test results of the multitude of test items in at least one matrix out of the matrices of the respective stages.

2. The system according to claim 1,
   wherein the first estimating unit includes a first AI unit configured to estimate the first state of the first complex system using artificial intelligence that has learned correspondence between a plurality of the first matrices and a plurality of the first states through machine learning.

3. The system according to claim 2,
   wherein the first AI unit includes artificial intelligence produced by machine learning of correspondence with the first states through image recognition of the first matrices that include the plurality of cells.

4. The system according to any one of claims 1 to 3,
   further comprising a first generating unit configured to generate the first matrix that reflects relationships between test results of a plurality of test items at the first timing of the first complex system in each of the plurality of cells included in the matrices of the respective stages.

5. The system according to any one of claims 1 to 4,
   further comprising a second generating unit configured to generate the first matrix where test results of the multitude of test items at the first timing of the first complex system are expanded into the plurality of cells using the large amount of correlation information for correlations between the test results of the multitude of test items in the matrices of the respective stages.

6. The system according to any one of claims 1 to 5,
   further comprising an output unit configured to output the first matrix in which the plurality of cells are disposed in two dimensions with the multitude of test items set on X and Y axes.

7. The system according to any one of claims 1 to 6,
   further comprising a second estimating unit configured to estimate transitions in a state of the first complex system based on displacements between the first matrix and a second matrix that is produced by converting test results of the multitude of test items at second timing when time has passed from the first timing, of the first complex system into a matrix based on the large amount of correlation information for correlations between test results of the multitude of test items in at least one matrix out of the matrices of the respective stages.

8. The system according to claim 7,
   wherein the storage includes transition matrices that include information on transitions in the large amount of correlation information between the matrices of the respective stages and reflect changes in state over time for the multitude of target complex systems, and
   the second estimating unit includes a unit configured to compare displacements from the first matrix to the second matrix and the transition matrices to verify a second state of the first complex system that has been estimated based on the second matrix.

9. The system according to claim 7 or 8,
wherein the second estimating unit includes a unit configured to estimate transitions in the state of the first complex system from the second timing onward.

10. The system according to any one of claims 7 to 9,
wherein the second estimating unit includes a second AI unit configured to estimate transitions in the state of the first complex system using artificial intelligence that has learned correspondence between displacements between the plurality of first matrices and the plurality of second matrices and changes in state over time in the multitude of target complex systems through machine learning.

11. The system according to any one of claims 1 to 10,
further comprising a unit configured to automatically generate the large amount of correlation information for correlations between the multitude of test items of each stage using replicas of the target complex systems.

12. The system according to any one of claims 1 to 11,
wherein the system is a preliminary examination system and the target complex systems are the human bodies.

13. A method that estimates a state of an individual complex system out of a multitude of target complex systems using a computer,
wherein the computer includes first storage that stores matrices of respective stages that include a large amount of correlation information for correlations between test results of a multitude of test items which suggest states of the multitude of target complex systems corresponding to stages in changes over time in the states of the multitude of target complex systems, the matrices including a plurality of cells including correlation information for correlations between test results of a plurality of test items; and
the method comprising causing the computer to execute a first estimating process that estimates a first state of a first complex system that is an individual complex system based on a first matrix produced by converting test results for the multitude of test items at first timing of the first complex system into a matrix based on the large amount of correlation information for correlations between the test results of the multitude of test items in at least one matrix out of the matrices of the respective stages.

14. The method according to claim 13,
wherein the first estimating process includes estimating the first state of the first complex system using artificial intelligence that has learned correspondence between a plurality of first matrices and a plurality of first states through machine learning.

15. The method according to claim 13 or 14,
further comprising causing the computer to execute a process that generates the first matrix that reflects relationships between test results of a plurality of test items at the first timing of the first complex system in each of the plurality of cells included in the matrices of the respective stages.

16. The method according to any one of claims 13 to 15,
further comprising causing the computer to execute a process that generates the first matrix where test results of the multitude of test items at the first timing of the first complex system are expanded into the plurality of cells using the large amount of correlation information for correlations between the multitude of test items in the matrices of the respective stages.

17. The method according to any one of claims 13 to 16,
further comprising causing the computer to execute a second estimating process that estimates transitions in a state of the first complex system based on displacements between the first matrix and a second matrix that is produced by converting test results of the multitude of test items at second timing when time has passed from the first timing, of the first complex system into a matrix based on the large amount of correlation information for correlations between test results of the multitude of test items in at least one matrix out of the matrices of the respective stages.

18. The method according to claim 17,
wherein the storage includes transition matrices that include information on transitions in the large amount of correlation information between the matrices of the respective stages and reflect changes in state over time in the multitude of target complex systems, and

the second estimating process includes comparing displacements from the first matrix to the second matrix and the transition matrices and verifying a second state of the first complex system that has been estimated based on the second matrix.

19. The method according to claim 17 or 18,
wherein the second estimating process includes estimating transitions in the state of the first complex system from the second timing onward.

20. A program for executing the method according to any one of claims 13 to 19 using a computer.

# Fig. 1

# Fig. 2

# Fig. 3

# Fig. 4

## Fig. 5

## Fig. 6

Fig. 7

Fig. 8

# Fig. 9

# Fig. 10

Fig. 11

# Fig. 12

# Fig. 13

# Fig. 14

(a)

**SN LDL vs TC**

13 — 131 — 139 — 138 — 136

(b)

**ND LDL vs TC**

13 — 132 — 139 — 138 — 136

(c)

**DS LDL vs TC**

13 — 133 — 139 — 138 — 136

# Fig. 15

(a)

LDL

135c
135b
135a

(b)

TC

135c
135b
135a

Fig. 16

# Fig. 17

(a)

(b)

Fig. 18

(a)

139 136

A·data N=554

TG

HDL

139   136

(b) 139 136

X·replica N=5540

TG

HDL

139   136

# Fig. 19

(a)

(b)

(c)

| LEVEL | 1 | 2 | 3 | 4 | 5 | 6 | 7 | 8 | 9 | 10 |
|---|---|---|---|---|---|---|---|---|---|---|
| FAVORABLE (BLUE) | | | | | | | | | | |
| UNFAVORABLE (RED) | | | | | | | | | | |

# Fig. 20

Fig. 21

Fig. 22

Fig. 23

START — 500

DETERMINE STAGE TO WHICH HEALTH STATE OF PATIENT BELONGS — 510

GENERATE INDIVIDUAL MATRIX — 520

ESTIMATE HEALTH STATE OF PATIENT — 530

GENERATE INDIVIDUAL DISPLACEMENT MATRIX — 540

ESTIMATE TRANSITION IN HEALTH STATE OF PATIENT — 550

OUTPUT ESTIMATION RESULT — 560

END

Fig. 24

## INTERNATIONAL SEARCH REPORT

| International application No. |
| --- |
| PCT/JP2019/004812 |

**A. CLASSIFICATION OF SUBJECT MATTER**
Int.Cl. G06N20/00(2019.01)i, G06F16/90(2019.01)i, G06Q50/22(2018.01)i

According to International Patent Classification (IPC) or to both national classification and IPC

**B. FIELDS SEARCHED**

Minimum documentation searched (classification system followed by classification symbols)
Int.Cl. G06N20/00, G06F16/90, G06Q50/22

Documentation searched other than minimum documentation to the extent that such documents are included in the fields searched
| | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2007-058565 A (TOTTORI UNIVERSITY) 08 March 2007, paragraphs [0015]-[0040], fig. 5 (Family: none) | 1-20 |
| Y | WO 2014/178323 A1 (ADVANCED TELECOMMUNICATIONS RESEARCH INSTITUTE INTERNATIONAL) 06 November 2014, paragraphs [0107]-[0141], [0170] & US 2015/0272461 A1, paragraphs [0141]-[0177], [0208] & EP 2992824 A1 & CN 105163659 A | 1-20 |

☒ Further documents are listed in the continuation of Box C.    ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 19 April 2019 (19.04.2019) | 07 May 2019 (07.05.2019) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

International application No.

PCT/JP2019/004812

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2017-220222 A (FUJITSU LTD.) 14 December 2017, paragraphs [0005]-[0026] & EP 3255586 A1, paragraphs [0004]-[0025] | 3-12, 18-20 |
| Y | JP 2004-130142 A (SAMSUNG ELECTRONICS CO., LTD.) 30 April 2004, paragraphs [0083]-[0084], [0108]-[0110] & US 2004/0117212 A1, paragraphs [0109]-[0110], [0134]-[0136] & EP 1407713 A1 & KR 10-2004-0032451 A | 8-12, 18-20 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017091586 A